# EUROPEAN PATENT SPECIFICATION

(11) **EP 1 175 397 B1**
(45) Date of publication and mention of the grant of the patent: **18.08.2004**
(21) Application number: 00930201.9
(22) Date of filing: 01.05.2000
(51) Int. Cl.: C07D 209/08, A61K 31/404, A61P 5/36

(54) **3,3-SUBSTITUTED INDOLINE DERIVATIVES**
3,3-SUBSTITUERTE INDOLINDERIVATE
DERIVES DE L'INDOLINE A SUBSTITUTION 3,3

(30) Priority: 04.05.1999 US 183061 P; 19.04.2000 US 552352
(43) Date of publication of application: 30.01.2002
(73) Proprietor: Wyeth, Madison, New Jersey 07940 (US); LIGAND PHARMACEUTICALS, INC., San Diego California 92121-1117 (US)
(72) Inventor: ULLRICH, John, W., Schwenksville, PA 19743 (US); FENSOME, Andrew, Wayne, PA 19087 (US); ZHI, Lin, San Diego, CA 92129 (US); JONES, Todd, K., Solana Beach, CA 92075 (US); WROBEL, Jay, E., Lawrenceville, NJ 08648 (US); TEGLEY, Christopher, M., Thousand Oaks, CA 91360 (US); EDWARDS, James, P., San Diego, CA 92129 (US)
(74) Representative: Hale, Stephen Geoffrey
(86) International application number: PCT/US2000/011464
(87) International publication number: WO 2000/066554

(56) References cited:
- EP-A- 0 535 529
- WO-A-94/14434
- WO-A-98/27059
- US-A- 5 688 810
- US-A- 5 696 127
- KATO L. PERLMAN ET AL.: "20-Oxopregnacalciferols: Vitamin D compounds that bind the Progesterone Receptor" TETRAHEDRON LETTERS, vol. 35, no. 15, 1994, pages 2295-2298, XP002029720 great Britain cited in the application

## Description

### Field of the Invention

This invention relates to compounds which are antagonists of the progesterone receptor, their preparation and utility.

### Background of the Invention

Intracellular receptors (IR) form a class of structurally related gene regulators known as "ligand dependent transcription factors" (R. M. Evans, *Science,* **240**, 889, 1988). The steroid receptor family is a subset of the IR family, including progesterone receptor (PR), estrogen receptor (ER), androgen receptor (AR), glucocorticoid receptor (GR), and mineralocorticoid receptor (MR).

The natural hormone, or ligand, for the PR is the steroid progesterone, but synthetic compounds, such as medroxyprogesterone acetate or Ievonorgestrel, have been made which also serve as ligands. Once a ligand is present in the fluid surrounding a cell, it passes through the membrane *via* passive diffusion, and binds to the IR to create a receptor/ligand complex. This complex binds to specific gene promoters present in the cell's DNA. Once bound to the DNA the complex modulates the production of mRNA and protein encoded by that gene.

A compound that binds to an IR and mimics the action of the natural hormone is termed an agonist, whilst a compound which inhibits the effect of the hormone is an antagonist.

PR antagonists may be used in contraception. In this context they may be administered alone (Ulmann, et al, *Ann*. *N.Y. Acad. Sci.,* **261**, 248, 1995), in combination with a PR agonist (Kekkonen, et al, *Fertility and Sterility,* **60**, 610, 1993) or in combination with a partial ER antagonist such as tamoxifen (WO 96/19997, published July 4, 1996).

PR antagonists may also be useful for the treatment of hormone dependent breast cancers (Horwitz, et al, Horm. Cancer, 283, pub: Birkhaeuser, Boston, Mass., ed. Vedeckis) as well as uterine and ovarian cancers. PR antagonists may also be useful for the treatment of non-malignant chronic conditions such as fibroids (Murphy, et al, *J. Clin. Endo. Metab*., **76**, 513, 1993) and endometriosis (Kettel, et al, *Fertility and Sterility*, **56**, 402, 1991). PR antagonists may further be useful in hormone replacement therapy for post menopausal patients in combination with a partial ER antagonist such as tamoxifen (U.S. Patent No. 5,719,136). PR antagonists, such as mifepristone and onapristone, have been shown to be effective in a model of hormone dependent prostate cancer, which may indicate their utility in the treatment of this condition in men (Michna, et al, *Ann. N.Y. Acad. Sci.,* ***761****, 224, 1995)*.

Jones, *et al*, (U.S. Patent No. 5,688,810) describe the PR antagonist dihydroquinoline **A**.

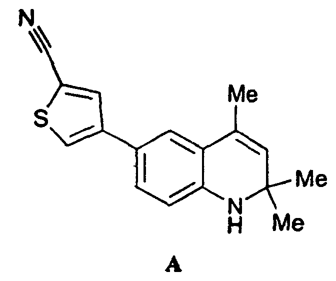

Jones, *et al*, described the enol ether **B** (U.S. Patent No. 5,693,646) as a PR ligand.

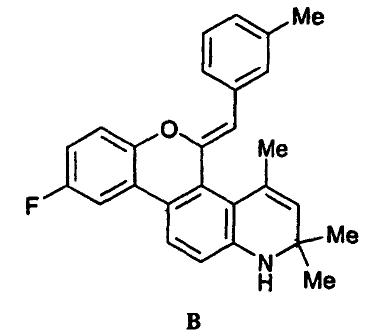

Jones, *et al*, described compound **C** (U.S. Patent No. 5,696,127) as a PR ligand.

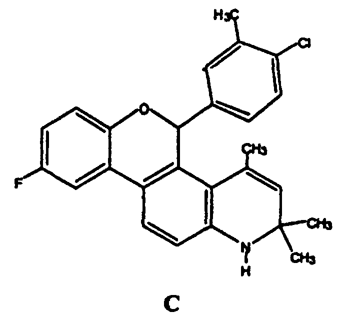

Zhi, *et al*, described lactones **D, E** and **F** as PR antagonists (J. Med. Chem., 41, 291, 1998).

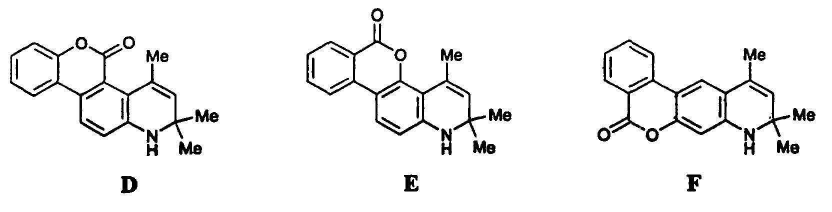

Zhi, *et al*, described the ether **G** as a PR antagonist (*J*. *Med. Chem*., **41**, 291, 1998).

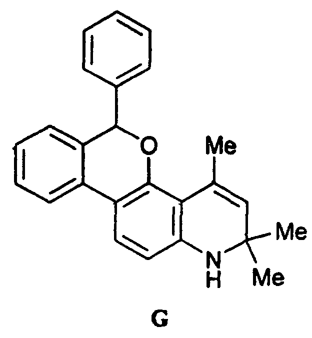

Combs, *et al*., disclosed the amide **H** as a ligand for the PR (*J. Med. Chem*., **38**, 4880, 1995).

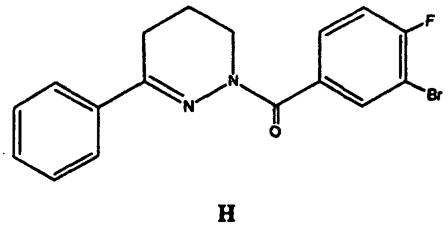

Perlman, *et. al*., described the vitamin D analog **I** as a PR ligand (*Tet. Letters*, **35**,2295,1994).

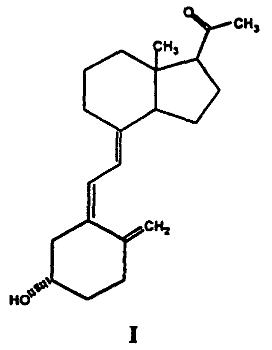

Hamann, *et al*, described the PR antagonist **J** (*Ann. N*.*Y*. *Acad*. *Sci.,* **761**, 383, 1995).

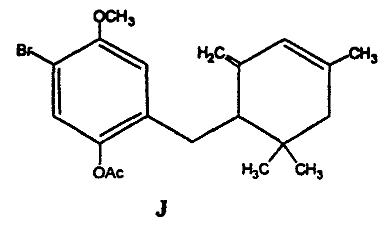

Chen, *et al*, described the PR antagonist **K** (Chen, et al, POI-37, 16^{th} Int. Cong. Het. Chem., Montana, 1997).

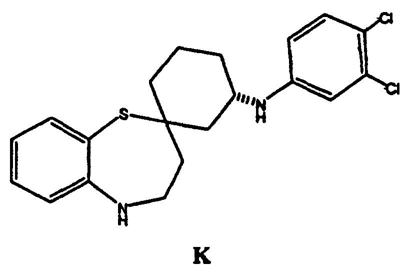

Kurihari, *et. al*., described the PR ligand **L** (*J. Antibiotics,* **50**, 360, 1997).

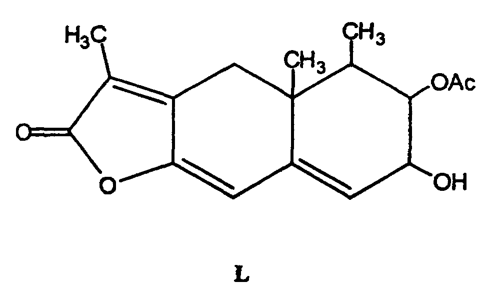

Elliott (Smith Kline Beecham) claimed the generic indoline **M** as potential endothelin receptor antagonists (WO 94/14434). The patent does not claim indolines and lacks the appropriate 5-aryl substitution, i.e. CN and NO₂. 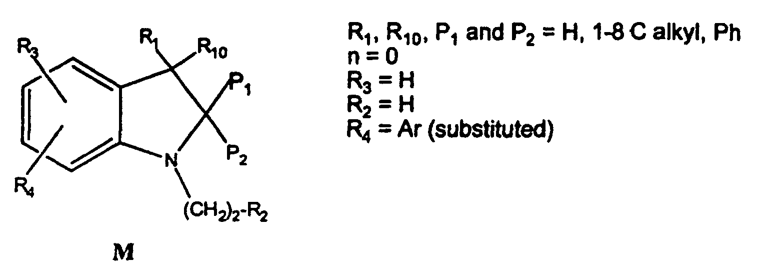 wherein: R₄ = H, Ar, R₁₁, OH, 1-5 C alkoxy (opt. substd. by OH, OMe or halogen), -S(O)_{q}R₁₁, N(R₆)₂, XR₁₁, halogen or NHCOR₆; X = (CH₂)ₙ, O, NR₆ or S(O)_{q}; n = 0-6; q = 0-2; R₆ = H or 1-4 C alkyl; R₁₁ = 1-8 C alkyl, 2-8 C alkenyl or 2-8 C alkynyl (all optionally substituted); Ar = (i) opt. substd. phenyl or benzo-fused gp. of (a) or (b); or (ii) napthyl, indoyl, pyridyl, thienyl, oxazolindyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, pyrrolyl or pyrimidyl, all opt. substd. by one or more R₁ or R₂ groups.

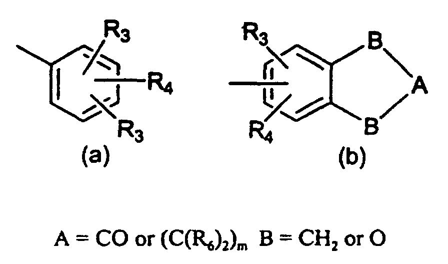

WO 98/27059 discloses indolone derivatives having an inhibitory activity on the bonding of progesterone to its receptor.

### Description of the Invention

The compounds of this invention have been shown to act as competitive inhibitors of progesterone binding to the PR and act as antagonists in functional models, either/or *in-vitro* and *in*-*vivo*. These compounds may be used for contraception, in the treatment and/or prevention of fibroids, including uterine fibroids, endometriosis, breast, uterine, ovarian and prostate cancer, and post menopausal hormone replacement therapy. This invention also particularly relates to methods of using these compounds in the inducement of contraception and the treatment and/or prevention of benign and malignant neoplastic disease. Such diseases may include, without limitation, benign prostatic hypertrophy, carcinomas and adenocarcinomas of the endometrium, ovary, breast, colon, prostate, pituitary, meningioma and other hormone-dependent tumors.

Compounds of this invention include compounds of the formula **I**: 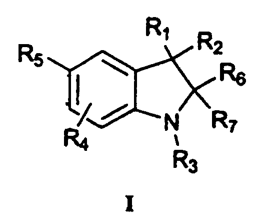 wherein:
R₁ and R₂ are chosen independently from H, alkyl, substituted alkyl; OR; O(alkyl); O(substituted alkyl); OAc; aryl; optionally substituted aryl; heteroaryl; optionally substituted heteroaryl; alkylaryl; alkylheteroaryl; 1-propynyl; or 3-propynyl;
or R₁ and R₂ are joined to form a ring comprising one of the following:
   -CH₂(CH₂)ₙCH₂-; -CH₂CH₂CMe₂CH₂CH₂-; -O(CH₂)ₘCH₂-; O(CH₂)ₚO-; -CH₂CH₂OCH₂CH₂-; -CH₂CH₂N(H or alkyl)CH₂CH₂-;
n is an integer from 0 to 5;
m is an integer from 1 to 4;
p is an integer from 1 to 4;
or R₁ and R₂ together comprise a double bond to =C(CH₃)₂; =C(C₃-C₆ cycloalkyl), =O, or =C(cycloether), wherein cycloether is selected from tetrahydrofuranyl or hexahydropyranyl;
R₃ is H, OH, NH₂, C₁ to C₆ alkyl, substituted C₁ to C₆ alkyl, C₃ to C₆ alkenyl, alkynyl or substituted alkynyl, or COR^{A};
R^{A} = H, C₁ to C₃ alkyl, substituted C₁ to C₃ alkyl, C₁ to C₃ alkoxy, substituted C₁ to C₃ alkoxy, C₁ to C₃ aminoalkyl, or substituted C₁ to C₃ aminoalkyl;
R₄ = H, halogen, CN, NH₂, C₁ to C₆ alkyl, substituted C₁ to C₆ alkyl, C₁ to C₆ alkoxy, substituted C₁ to C₆ alkoxy, C₁ to C₆ aminoalkyl, or substituted C₁ to C₆ aminoalkyl;
R₅ is selected from the groups a), b) or c):
   a) R₅ is a trisubstituted benzene ring containing the substituents X, Y and Z as shown below: 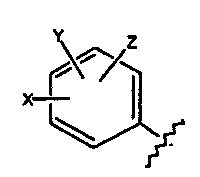
      X is selected from halogen, OH, CN, C₁ to C₃ alkyl, substituted C₁ to C₃ alkyl, C₁ to C₃ alkoxy, substituted C₁ to C₃ alkoxy, C₁ to C₃ thioalkyl, substituted C₁ to C₃ thioalkyl, S(O)alkyl, S(O)₂alkyl, C₁ to C₃ aminoalkyl, substituted C₁ to C₃ aminoalkyl, NO₂, C₁ to C₃ perfluoroalkyl, 5 or 6 membered heterocyclic ring containing 1 to 3 heteroatoms, COR^{B}, OCOR^{B}, or NR^{C}COR^{B};
      R^{B} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
      R^{C} is H, C₁ to C₃ alkyl, or substituted C₁ to C₃ alkyl:
      Y and Z are independent substituents taken from the group including H, halogen, CN, NO₂, C₁ to C₃ alkoxy, C₁ to C₃ alkyl, or C₁ to C₃ thioalkyl; or
   b) R₅ is a five or six membered ring with 1, 2, or 3 heteroatoms from the group including O, S, SO, SO₂ or NR₈ and containing one or two independent substituents from the group including H, halogen, CN, NO₂ and C₁ to C₃ alkyl, C₁ to C₃ alkoxy, C₁ to C₃ aminoalkyl, COR^{D}, or NR^{E}COR^{D};
      R^{D} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
      R^{E} is H, C₁ to C₃ alkyl, or substituted C₁ to C₃ alkyl;
      R₈ is H or C₁ to C₃ alkyl;
      or
   c) R⁵ is an indol-4-yl, indol-7-yl or benzo-2-thiophene moiety, the moiety being optionally substituted by from 1 to 3 substituents selected from halogen, lower alkyl, CN, NO₂, lower alkoxy, or CF₃; wherein R₆ and R₇ are independently chosen from H, methyl, ethyl, propyl, butyl, iso-propyl, iso-butyl, cyclohexyl, aryl, substituted aryl, heteroaryl, or substituted heteroaryl;
or pharmaceutically acceptable salt thereof.

A preferred set of compounds of this invention is depicted by structure **II**: 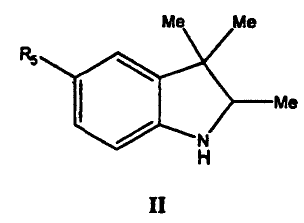 wherein:
R₅ is a disubstituted benzene ring containing the substituents X, and Y as shown below: 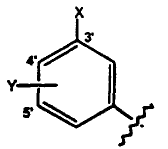
X is selected from halogen, CN, C₁ to C₃ alkoxy, C₁ to C₃ alkyl, NO₂, C₁ to C₃ perfluoroalkyl, 5-membered heterocyclic ring containing 1 to 3 heteroatoms, or C₁ to C₃ thioalkoxy;
Y is a substituent on the 4' or 5'position selected from H, halogen, CN, NO₂, C₁ to C₃ alkoxy, C₁ to C₄ alkyl, or C₁ to C₃ thioalkyl;
   or
R₅ is a five membered ring having the structure: 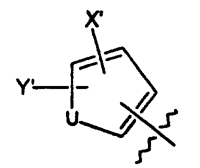
U is O, S, or NR_{6'}
R_{6'} is H, or C₁ to C₃ alkyl, or C₁ to C₄ CO₂alkyl;
X' is selected from halogen, CN, NO₂, C₁ to C₃ alkyl or C₁ to C₃ alkoxy;
Y' is selected from the group H, F, CN, NO₂ or C₁ to C₄ alkyl;
   or
R₅ is a six-membered ring with the structure: 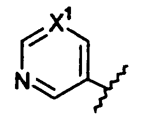
X¹ is N or CX²;
X² is halogen, CN or NO₂;
or pharmaceutically acceptable salt thereof.

The compounds of this invention may contain an asymmetric carbon atom and some of the compounds of this invention may contain one or more asymmetric centers and may thus give rise to optical isomers and diastereomers. While shown without respect to stereochemistry in Formulas I and II, the present invention includes such optical isomers and diastereomers; as well as the racemic and resolved, enantiomerically pure R and S stereoisomers; as well as other mixtures of the R and S stereoisomers and pharmaceutically acceptable salts thereof.

The term "alkyl" is used herein to refer to both straight- and branched-chain saturated aliphatic hydrocarbon groups having 1 to 8 carbon atoms, preferably 1 to 6 carbon atoms; "alkenyl" is intended to include both straight- and branched-chain alkyl group with 1 or 2 carbon-carbon double bonds and containing 2 to 8 carbon atoms, preferably 2 to 6 carbon atoms; "alkynyl" group is intended to cover both straight- and branched-chain alkyl group with at least 1 or 2 carbon-carbon triple bonds and containing 2 to 8 carbon atoms, preferably 2 to 6 carbon atoms.

The terms "substituted alkyl", "substituted alkenyl", and "substituted alkynyl" refer to alkyl, alkenyl, and alkynyl as just described having one or more substituents from the group including halogen, CN, OH, NO₂, amino, aryl, heterocyclic, substituted aryl, substituted heterocyclic, alkoxy, aryloxy, substituted alkyloxy, alkylcarbonyl, alkykarboxy, alkylamino, arylthio. These substituents may be attached to any carbon of alkyl, alkenyl, or alkynyl group provided that the attachment constitutes a stable chemical moiety.

The term "aryl" is used herein to refer to an aromatic system which may be a single ring or multiple aromatic rings fused or linked together as such that at least one part of the fused or linked rings forms the conjugated aromatic system. The aryl groups include but not limited to phenyl, naphthyl, biphenyl, anthryl, tetrahydronaphthyl, phenanthryl.

The term "substituted aryl" refers to an aryl as just defined having 1 to 4 substituents from the group including halogen, CN, OH, NO₂, amino, alkyl, cycloalkyl, alkenyl, alkynyl, alkoxy, aryloxy, substituted alkyloxy, alkylcarbonyl, alkylcarboxy, alkylamino, or arylthio.

The term "heterocyclic" is used herein to describe a stable 4- to 7-membered monocyclic or a stable multicyclic heterocyclic ring which is saturated, partially unsaturated, or unsaturated, and which consists of carbon atoms and from one to four heteroatoms selected from the group including N, O, and S atoms. The N and S atoms may be oxidized. The heterocyclic ring also includes any multicyclic ring in which any of above defined heterocyclic rings is fused to an aryl ring. The heterocyclic ring may be attached at any heteroatom or carbon atom provided the resultant structure is chemically stable. Such heterocyclic groups include, for example, tetrahydrofuran, piperidinyl, piperazinyl, 2-oxopiperidinyl, azepinyl, pyrrolidinyl, imidazolyl, pyridyl, pyrazinyl, pyrimidinyl, pyridazinyl, oxazolyl, isoxazolyl, morpholinyl, indolyl, quinolinyl, thienyl, furyl, benzofuranyl, benzothienyl, thiamorpholinyl, thiamorpholinyl sulfoxide, and isoquinolinyl.

The term "substituted heterocyclic" is used herein to describe the heterocyclic just defined having 1 to 4 substituents selected from the group which includes halogen, CN, OH, NO₂, amino, alkyl, substituted alkyl, cycloalkyl, alkenyl, substituted alkenyl, alkynyl, alkoxy, aryloxy, substituted alkyloxy, alkylcarbonyl, alkylcarboxy, alkylamino, or arylthio. The term "thioalkyl" is used herein to refer to the SR group, where R is alkyl or substituted alkyl, containing 1 to 8 carbon atoms. The term "alkoxy" refers to the OR group, where R is alkyl or substituted alkyl, containing 1 to 8 carbon atoms. The term "aryloxy" refers to the OR group, where R is aryl or substituted aryl, as defined above. The term "alkylcarbonyl" refers to the RCO group, where R is alkyl or substituted alkyl, containing 1 to 8 carbon atoms, preferably 1 to 6 carbon atoms. The term "alkylcarboxy" indicates the COOR group, where R is alkyl or substituted alkyl, containing 1 to 8 carbon atoms, preferably 1 to 6 carbon atoms. The term "aminoalkyl" refers to both secondary and tertiary amines wherein the alkyl or substituted alkyl groups, containing 1 to 8 carbon atoms, which may be either the same or different and the point of attachment is on the nitrogen atom. The term "halogen" refers to Cl, Br, F, or I.

The compounds of the present invention can be used in the form of salts derived from pharmaceutically or physiologically acceptable acids or bases. These salts include, but are not limited to, the following salts with inorganic acids such as hydrochloric acid, sulfuric acid, nitric acid, phosphoric acid and, as the case may be, such organic acids as acetic acid, oxalic acid, succinic acid, and maleic acid. Other salts include salts with alkali metals or alkaline earth metals, such as sodium, potassium, calcium or magnesium in the form of esters, carbamates and other conventional "pro-drug" forms, which, when administered in such form, convert to the active moiety *in vivo*.

This invention includes pharmaceutical compositions comprising one or more compounds of this invention and a pharmaceutically acceptable carrier or excipient. The invention also includes methods of treatment which comprise administering to a mammal a pharmaceutically effective amount of one or more compounds as described above as antagonists of the progesterone receptor.

The progesterone receptor antagonists of this invention, used alone or in combination, can be utilized in methods of contraception and the treatment and/or prevention of benign and malignant neoplastic disease. Specific uses of the compounds and pharmaceutical compositions of invention include the treatment and/or prevention of uterine myometrial fibroids, endometriosis, benign prostatic hypertrophy; carcinomas and adenocarcinomas of the endometrium, ovary, breast, colon, prostate, pituitary, meningioma and other hormone-dependent tumors, particularly progesterone-related tumors. Additional uses of the present progesterone receptor antagonists include the synchronization of the estrus in livestock.

When the compounds are employed for the above utilities, they may be combined with one or more pharmaceutically acceptable carriers or excipients, for example, solvents, diluents and the like, and may be administered orally in such forms as tablets, capsules, dispersible powders, granules, or suspensions containing, for example, from about 0.05 to 5% of suspending agent, syrups containing, for example, from about 10 to 50% of sugar, and elixirs containing, for example, from about 20 to 50% ethanol, and the like, or parenterally in the form of sterile injectable solutions or suspensions containing from about 0.05 to 5% suspending agent in an isotonic medium. Such pharmaceutical preparations may contain, for example, from about 25 to about 90% of the active ingredient in combination with the carrier, more usually between about 5% and 60% by weight.

The effective dosage of active ingredient employed may vary depending on the particular compound employed, the mode of administration and the severity of the condition being treated. However, in general, satisfactory results are obtained when the compounds of the invention are administered at a daily dosage of from about 0.5 to about 500 mg/kg of animal body weight, preferably given in divided doses two to four times a day, or in a sustained release form. For most large mammals, the total daily dosage is from about 1 to 100 mg, preferably from about 2 to 80 mg. Dosage forms suitable for internal use comprise from about 0.5 to 500 mg of the active compound in intimate admixture with a solid or liquid pharmaceutically acceptable carrier. This dosage regimen may be adjusted to provide the optimal therapeutic response. For example, several divided doses may be administered daily or the dose may be proportionally reduced as indicated by the exigencies of the therapeutic situation.

These active compounds may be administered orally as well as by intravenous, intramuscular, or subcutaneous routes. Solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the standpoint of ease of preparation and administration are solid compositions, particularly tablets and hard-filled or liquid-filled capsules. Oral administration of the compounds is preferred.

These active compounds may also be administered parenterally or intraperitoneally. Solutions or suspensions of these active compounds as a free base or pharmacologically acceptable salt can be prepared in water suitably mixed with a surfactant such as hydroxypropylcellulose. Dispersions can also be prepared in glycerol, liquid, polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringe ability exits. It must be stable under conditions of manufacture and storage and must be preserved against the contaminating action of microorganisms such as bacterial and fungi. The carrier can be a solvent or dispersion medium containing, for example, water, ethanol (e.g., glycerol, propylene glycol and liquid polyethylene glycol), suitable mixtures thereof, and vegetable oil.

The compounds of this invention can be prepared by the procedures outlined in the Schemes illustrated below:

Typically the compounds of this invention are prepared in a convergent manner as shown is Scheme 1, by a suitable coupling reaction. For example, a palladium mediated coupling of an aryl halide with an aryl boronic acid provides the desired bi-aryl substituted target. The choice of the aryl halide-aryl boronic acid combination is established experimentally.

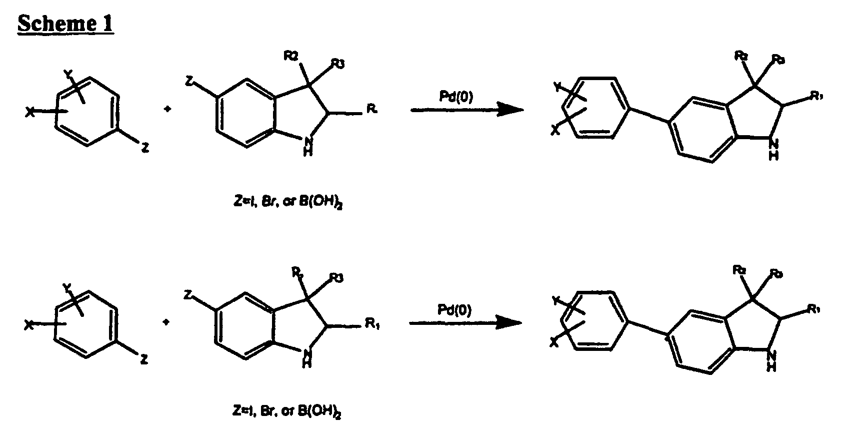

As outlined in **Scheme 2**, the "right side" of the compounds of this invention may be prepared by following the procedure described in Letcher, R. M. et. al., *J*. *Chem. Soc. Perkin Trans.,* **1**: 939 - 944, 1993.

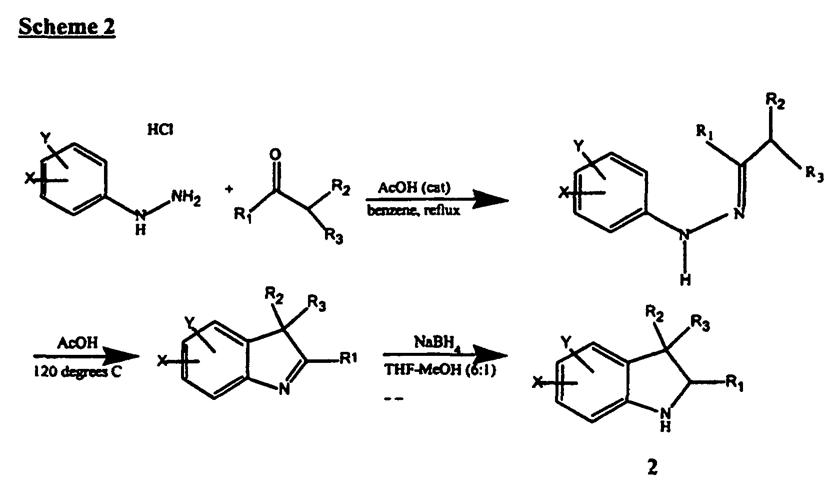

As an example, the right side template, **2**, is prepared by condensing an appropriately substituted phenyl hydrazine and a suitable ketone to give the corresponding hydrazone. This material is cyclized to an imine in refluxing acetic acid and then reduced to the desired indoline template **2**. Examples **1-7** and **10-20** were prepared by this route using the appropriate ketone.

Alternatively the right side template may be prepared as outlined in **Scheme 3**. The commercially available oxindole is di-alkylated at C-3 by using an appropriate base and the corresponding alkyl halide to give the 3,3-dialkyl-oxindole, **8**, or the spiro-cyclic oxindole **9**. These oxindoles are then brominated under standard conditions and the carbonyl group is reduced to the desired methylene using a hydride mediated reduction. The timing of the aryl coupling and the reduction of the 2-position carbonyl are established experimentally.

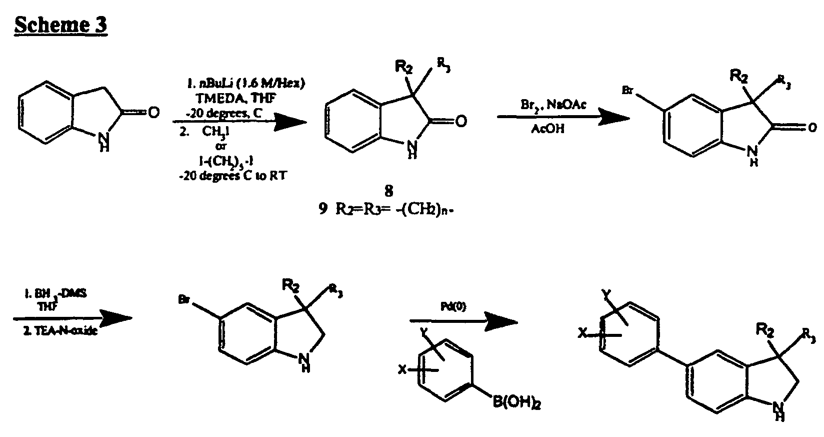

The right side templates are coupled with an appropriate aryl boronic acid using an appropriate palladium (0) catalyst, **Scheme 4**. For example compound **10** is coupled under standard Suzuki conditions with an appropriately substituted arylboronic acid to afford compound **11**.

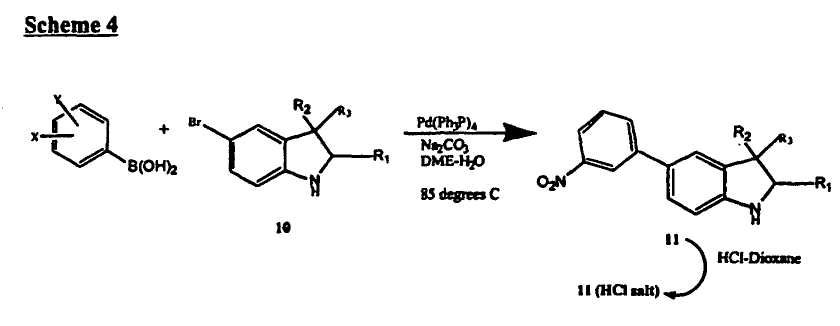

The compounds of this invention, are stable semi-solids and are conveniently converted into their corresponding salts by treatment with acid. Example **1**, compound **11** (R₁ = R₂ = R₃ = Me), when treated with HCl in dioxane affords the HCl salt (Example 2) as a white solid. The racemic indolines can be separated into their enantiomers by Chiral HPLC, to provide the individual enantiomers in > 98% EE.

This invention may be further understood by the following non-limiting examples.

### EXAMPLE 1

### 2,3,3-Trimethyl-5-(3-nitro-phenyl)-2,3-dihydro-1H-indole

### 5-Bromo-2,3,3-trimethyl-2,3-dihydro-1H-indole

This compound was prepared by the procedure of Letcher, R. M. et. al., *J. Chem. Soc. Perkin Trans*., **1**: 939 - 944,1993.

To a solution of 4-bromo-phenyl hydrazine hydrochloride (2.59g, 11.6 mmol) and 3-methyl-2-butanone (1.0 g, 11.6 mmol) in 20 mL benzene was added acetic acid (catalytic amount) and the resulting solution was refluxed with azeotropic removal of H₂O for 14 h. The reaction mixture was cooled to room temperature and concentrated. The resulting semi-solid was extracted in acetic acid and refluxed for 12 hours. The reaction mixture was cooled to room temperature and concentrated. The semi-solid residue was extracted in ether and neutralized with K₂CO₃. The ether layer was dried and concentrated. The resulting solid imine was dissolved in THF-MeOH (6:1), cooled to 0°C and sodium borohydride (0.5 g, 13.2 mmol) was added. The solution was allowed to warm to room temperature and stirred for 0.5 hours. The reaction mixture was poured into 15% aqueous HCl and then made basic with K₂CO₃. The organic layer was separated, washed with brine, dried (Na₂SO₄), and concentrated. The crude product was purified by column chromatography (SiO₂, hexane-ethyl acetate 9:1). The product was isolated as an orange liquid (2.1 g, 75%): ¹H-NMR (CDCl₃) δ 1.03 (s, 3H), 1.15 (d, *J* = 6.6 Hz, 3H), 1.25 (s, 3H), 3.50 (q, *J* = 6.6 Hz, 1H), 3.70 (br, s, 1H), 6.45 (d, *J* = 8.7 Hz, 1H), 7.09 (m, 2H); ¹³C-NMR (CDCl₃) δ 15.10, 22.25, 26.11 (q), 43.72 (s), 65.47 (d), 110.32 (s), 110.70, 125.47, 129.75 (d), 141.48, 148.33 (s); MS (EI*) m*/*z* 240, 242 (M+H)⁺.

A solution of 5-bromo-2,3,3-trimethyl-2,3-dihydro-1H-indole (0.5 g, 2.1 mmol) and tetrakis-(triphenylphosphine) palladium (0.14 g, 0.12 mmol) in dimethoxyethane (10 mL) was cycled under N₂-vacuum (4x) and then stirred under N₂ for 0.5 hours. To this mixture was then added 3-nitrophenylboronic acid (0.42 g, 2.5 mmol) followed by a solution of Na₂CO₃ (0.36 g, 3.4 mmol) in 5 mL water cycled under N₂-vacuum (3x). The solution was brought to reflux for 6 hours then cooled to room temperature, poured into water and extracted with EtOAc. The combined organic extracts were washed with water, brine, dried (Na₂SO₄), and evaporated. The residue was purified by column chromatography (SiO₂, methylenechloride:hexane 1:3) to afford the title compound (0.48 g, 82%) as an orange semi-solid: ¹H NMR (CDCl₃) δ 1.12 (s, 3H), 1.21 (d, *J* = 6.6 Hz, 3H), 1.35 (s, 3H), 3.60 (q, *J* = 6.6 Hz, 1H), 3.9 (br s, 1H), 6.69 (d, *J* = 7.9 Hz, 1H), 7.28-7.29 (m, 2H), 7.32 (d, *J* = 1.9 Hz, 1H), 7.53 (dd, *J* = 7.9, 7.9 Hz, 1H), 7.85 (ddd, *J* = 7.9, 2.0, 2.0 Hz, 1H), 8.07 (ddd, *J* = 7.9, 2.0, 2.0 Hz, 1H), 8.08 (d, *J* = 7.9 Hz, 1H), 8.39 (dd *J* = 2.0, 2.0 Hz, 1H); ¹³C-NMR (CDCl₃) δ 15.27, 22.54, 26.39 (q), 43.53 (s), 65.57 (d), 109.48, 120.67, 121.04, 121.10, 126.58 (d), 129.15 (s), 129.53, 132.35 (d), 140.18, 143.64, 148.78, 150.11 (s); MS (EI*) m*/*z* 283 (M+H)⁺.

### EXAMPLE 2

### 2,3,3-Trimethyl-5-(3-nitro-phenyl)-2,3-dihydro-1H-indole Hydrochloride

A solution of 2,3,3-trimethyl-5-(3-nitro-phenyl)-2,3-dihydro-1H-indole (0.8 g, 2.79 mmol) in 20 mL of 1:1 ether:dioxane at room temperature was treated with 1.5 mL of a 4 M HCl/dioxane solution. The resulting solid was isolated by filtration and washed with hexane to afford the title compound (0.81 g, 90%) as a tan solid: m.p. 240-241 °; ¹H-NMR (CDCl₃) δ 1.37 (s, 3H), 1.51 (s, 3H), 4.01 (d, *J =* 6.5 Hz, 1H), 7.51 (s, 1H), 7.59 (d, *J* = 7.3 Hz, 1H), 7.7 (dd, *J* = 7.9, 7.9 Hz, 1H), 7.8 (d, *J* = 7.9, Hz, 1H), 7.89 (d, *J* = 7.7 Hz, 1H), 8.3 (dd, *J* = 7.9, 1.8 Hz, 1H), 8.42 (s, 1H), 11.9 (br s. 2H); ¹³C-NMR (CDCl₃) δ; MS (EI*) m*/*z* 283 (M+H)⁺.

### EXAMPLE 3

### (2-R or S)-2,3,3-Trimethyl-5-(3-nitro-phenyl)-2,3-dihydro-1H-indole and

### and

### EXAMPLE 4

### (2-S or R)-2,3,3-Trimethyl-5-(3-nitro-phenyl)-2,3-dihydro-1H-indole

A sample of racemic 2,3,3-trimethyl-5-(3-nitro-phenyl)-2,3-dihydro-1H-indole (25 mg), as prepared in example 2, was separated by a chiral preparative HPLC [column: Chiralcel OD, 4.6 X 250 mm, isocratic, 5:95 IPA:hexane, flow rate = 1 mL/min; injection volume = 5 µL; retention times: **3**, 9.2 min and **4**, 10.39 min.] to provide the enantiomers **3** and **4** as orange semi-solids. Chiral Analytical HPLC determined that the enantiomers had > 99 % EE. Examples **3** and **4** have spectral data identical to the racemic material, example 1 : ¹H- NMR (CDCl₃) δ 1.12 (s, 3H), 1.21 (d, *J* = 6.6 Hz, 3H), 1.35 (s, 3H), 3.60 (q, *J* = 6.6 Hz, 1H), 3.9 (br s, 1H), 6.69 (d, *J =* 7.9 Hz, 1H), 7.28-7.29 (m, 2H), 7.32 (d, *J =* 1.9 Hz, 1H), 7.53 (dd, *J* = 7.9, 7.9 Hz, 1H), 7.85 (ddd, *J =* 7.9, 2.0, 2.0 Hz, 1H), 8.07 (ddd, *J* = 7.9, 2.0, 2.0 Hz, 1H), 8.08 (d, *J =* 7.9 Hz, 1H), 8.39 (dd, *J =* 2.0, 2.0 Hz, 1H); MS (EI*) m*/*z* 283 (M+H)⁺.

### EXAMPLE 5

### 2,3,3-Diethyl-2-methyl-5-(3-nitro-phenyl)-2,3-dihydro-1H-indole

### 5-Bromo-3,3-diethyl-2-methyl-2,3-dihydro-1H-indole

This compound was prepared according to the procedure for Example 1 using 4-bromo-phenyl hydrazine hydrochloride (5.9 g, 26.3 mmol) and 3-ethyl-2-pentanone (3.0 g, 26.3 mmol). The subtitled compound (4.5 g) was obtained in 65 % yield as a yellowish oil: ¹H-NMR (CDCl₃) δ 0.81 (t, *J* = 7.4 Hz, 3H), 0.83 (t, *J* = 7.4 Hz, 3H), 1.18 (d, *J* = 6.5 Hz, 3H), 1.42 (dq, *J* = 14.0, 7.4 Hz, 1H), 1.66 (dq, J = 14.0, 7.4 Hz, 3H), 3.73 (q, *J* = 6.5 Hz, 1H), 6.47 (d, *J* = 8.2 Hz, 1H), 7.02 (d, *J* = 2 Hz, 1H), 7.09 (dd, *J* = 8.2, 2.0 Hz, 1H); ¹³C-NMR (CDCl₃) δ 10.46, 10.66, 17.70 (q), 26.5, 29.67 (t), 52.25 (s), 64.80 (d), 111.64 (s), 112.41, 129.07,131.58 (d), 139.57, 151.04 (s); MS (EI*) m*/*z* 268, 270 (M+H)⁺.

Using the standard coupling conditions given in Example I the title compound was prepared from 5-bromo-3,3-diethyl-2-methyl-2,3-dihydro-1H-indole (0.13 g, 0.45 mmol), tetrakis-(triphenylphosphine) palladium (0.05 g, 0.04 mmol) in dimethoxyethane (4 mL) with 3-nitropbenylboronic acid (0.09 g, 0.54 mmol) and sodium carbonate (0.15 g, 4.95 mmol) in 2 mL of water. The title compound (0.09 g, 65 %) was obtained as a red brown glass: ¹H NMR (CDCl₃) δ 0.86 (dt, *J* = 4.0, 4.0 Hz, 3H), 0.88 (dt, *J* = 4.0, 4.0 Hz, 3H), 1.24 (d, *J* = 6.6 Hz, 3H), 1.5 (dq, *J =* 14.1, 7.3 Hz, 1H), 1.66-1.84 (m, 3H), 3.81 (q, *J* = 6.6 Hz, 1H), 6.69 (d, *J* = 8.0 Hz, 1H), 7.20 (d, *J* = 1.9 Hz, 1H), 7.32 (dd, *J* = 8.0, 1.9 Hz, 1H), 7.53 (dd, *J* = 8.0, 8.0 Hz, 1H), 7.85 (dd, *J* = 7.9, 1.1 Hz, 1H), 8.09 (dd, *J* = 7.9, 1.1 Hz, 1H), 8.4 (dd, *J* = 1.9 Hz, 1H); ¹³C-NMR (CDCl₃) δ 8.99, 9.14, 16.24 (q), 24.87, 28.14 (t), 50.30 (s), 63.31 (d), 109.56, 120.80, 121.22, 123.19, 126.76 (d), 128.64 (s), 129.67, 132.55 (d), 136.5, 143.92, 148.95, 151.07 (s); MS (EI*) m*/*z* 310 (M)⁺.

### EXAMPLE 6

### 4a-Methyl-6-(3-nitro-phenyl)-2,3,4,4a,9,9a-hexahydro-1H-carbazole

### 6-Bromo-4a-methyl-2,3,4,4a,9,9a-hexahydro-1H-carbazole

This compound was prepared by the procedure of Example 1 using 4-bromo-phenyl hydrazine hydrochloride (2.0 g, 8.95 mmol) and 2-methylcyclohexanone (1.0 g, 8.95 mmol). The subtitled compound (1.5 g, 65%) was obtained as a yellow oil: ¹H NMR (CDCl₃) δ 1.26 (s, 3H), 1.38-1.46 (m, 4H), 1.56-1.68 (m, 4H), 3.4 (t, *J*= 4.4 Hz, 1H), 3.6 (br s, 1H), 6.53 (d, *J* = 8 Hz, 1H), 7.08 (d, *J =* 2.0 Hz, 1H), 7.09 (dd, J = 8.0, 2.0 Hz, 1H); ¹³C-NMR (CDCl₃) δ 21.45, 21.87 (t), 23.96 (q), 27.92, 35.32 (t), 43.56 (s), 66.65 (d), 110.73 (s), 111.88, 125.25, 129.99 (d), 142.17, 149.03 (s); MS (EI*) m*/*z* 268, 270 (M+H)⁺.

Using the standard coupling conditions given in Example 1, the title compound was prepared using 6-bromo-4a-methyl-2,3,4,4a,9,9a-hexahydro-1H-carbazole (1.6 g, 6.0 mmol), tetrakis(triphenylphosphine) palladium (0.4 g, 0.35 mmol) in dimethoxyethane (30 mL) with 3-nitrophenylboronic acid (1.2 g, 7.2 mmol) and sodium carbonate (1.9 g, 18 mmol) in 10 mL water. The pure product (1.2 g, 70%) was obtained as an orange foam: ¹H NMR (CDCl₃) δ 1.36 (s, 3H), 1.44-1.74 (m, 8H), 3.49 (t, *J* = 4.4 Hz, 1H), 3.83 (br s, 1H), 6.75 (d, *J* = 8.0 Hz, 1H), 7.26 (d, *J =* 1.9 Hz, 1H), 7.32 (dd, *J =* 8.0, 1.9 Hz, 1H), 7.53 (dd, *J =* 8.0, 8.0 Hz, 1H), 7.86 (d, *J* = 7.8 Hz, 1H), 8.08 (dd, *J* = 8.0, 2.0 Hz, 1H), 8.39 (dd, *J* = 2.0, 2.0 Hz, 1H); ¹³C-NMR (CDCl₃) δ 21.27,21.71 (t), 23.87 (q), 27.76, 35.29 (t); 43.06 (s), 66.46 (d), 110.41, 120.57, 120.83, 121.24, 126.55 (d), 129.26 (s), 129.68, 132.55 (d), 140.66, 143.86, 148.95, 150.51 (s); MS (EI*) m*/*z* 309 (M+H)⁺.

### EXAMPLE 7

### 1,2-Dihydro-2-methyl-5-(3-nitro-phenyl)spiro[cyclohexane-1,3-[3H]indole]

### 5-Bromo-1,2-dihydro-2-methylspiro[cyclohexane-1,3-[3H] indole]

Using the conditions given in Example 1 the subtitled compound was prepared from 4-bromo-phenyl hydrazine HCl (3.5 g, 15.7 mmol) and cyclohexyl methyl ketone (2.0 g, 15.7 mmol). The pure material (3.0 g, 68%) was obtained as an oil: ¹H NMR (CDCl₃) δ 1.09 (d, *J =* 6.5 Hz, 3H), 135-1.73 (m, 10H), 3.45 (br s, 1H), 3.71 (q, *J =* 6.5 Hz, 1H), 6.47 (d, *J* = 8.2 Hz, 1H), 7.09, dd, *J* = 8.2, 2.0 Hz, 1H), 7.19 (d, *J* = 2.0 Hz, 1H); ¹³C-NMR (CDCl₃) δ 17.22 (q), 23.19, 23.44, 26.16, 30.17, 36.70 (t), 47.99 (s), 62.34 (d), 110.08 (s), 111.07, 126.83, 130.08 (d), 139.98, 148.49 (s); MS (EI*) m*/*z* 280, 282 (M+H)⁺.

Using the standard coupling conditions given in Example 1 the title compound was prepared from 5-bromo-1,2-dihydro-2-methylspiro[cyclohexane-1,3-[3H] indole] (0.5 g, 1.65 mmol), tetrakis(triphenylphosphine) palladium (0.08 g, 0.07 mmol) in dimethoxy-ethane (5 mL) with 3-nitrophenylboronic acid (0.33 g, 1.98 mmol) and sodium carbonate (0.53 g, 4.95 mmol) in 5 mL water. The pure material (0.35 g, 55%) was obtained as an orange brown semi-solid: ¹H NMR (CDCl₃) δ 1.16 (d, *J* = 6.4 Hz, 3H), 1.38-1.83 (m, 10H), 3.7 (br s, 1H), 3.8 (q, *J* = 6.4 Hz, 1H), 6.69 (d, *J* = 8.0 Hz, I H), 7.31 (dd, *J* = 8.0, 1.8 Hz, 1H), 7.53 (dd, *J* = 8.0, 8.0 Hz, 1H), 7.86 (d, *J* = 7.3 Hz, 1H), 8.09 (d, *J* = 8.0 Hz, 1H), 8.39. (dd, *J* = 1.9, 1.9 Hz, 1H); ¹³C-NMR (CDCl₃) δ 17.45 (q), 23.32, 23.70, 26.21, 30.31, 37.07 (t), 47.77 (s), 62.11 (d), 109.81, 120.78, 121.23, 122.40, 126.92 (d), 128.96 (s), 129.66, 132.51 (d), 138.63, 143.92, 148.94, 150.1 (s); MS (EI*) m*/*z* 322 (M)⁺.

### EXAMPLE 8

### 3,3-Dimethyl-5-(3-nitro-phenyl)-2,3-dihydro-1H-indole (WAY-160655)

### 3,3-dimethyl-1,3-dihydro-2H-indol-2-one

This compound was prepared using the general method described by A. Kende, *Synth. Commun*., **1**: 12 (1982). The crude material was purified by column chromatography (SiO₂, methylenechloride:hexane 1:3) to afford the subtitled compound which was consistent with the reported spectral data.

The above oxindole (0.65 g, 4.03 mmol) and sodium acetate (0.334 g, 4.07 mmol) were stirred in acetic acid (5.0 mL). Bromine (0.66 g, 0.00413 mol) in acetic acid (5.0 mL) was added drop-wise to the reaction mixture. The reaction was stirred for 50 minutes, and then poured into water (10 mL). The mixture was made basic with sodium carbonate, extracted with ethyl acetate, dried (MgSO₄), filtered, and evaporated to give 5-bromo-1,3-dihydro-3,3-dimethyl-2H-indol-2-one ( 0.89 g, 92%): ¹H NMR (DMSO-d₆) 1.21 (s, 6 H), 6.76 (d, *J*= 8.22 Hz, 1H), 7.29 (dd, *J*= 2.1, 8.2 Hz, 1H), 7.49 (d, *J =* 2.0 Hz, 1H), 10.4 (s, 1H).

To a solution of the 5-bromo-1,3-dihydro-3,3-dimethyl-2H-indol-2-one (0.9 g, 3.7 mmol) in 20 mL THF at 0 °C was added a borane-methylsulfide complex (2M in THF, 38 mL, 75 mmol). The reaction mixture was warmed to room temperature then brought to reflux for 4 hours. The mixture was cooled to room temperature and poured into H₂O/CH₂Cl₂ and washed with 5% NaHCO₃. The organic layer was washed with brine, dried (Na₂SO₄) and concentrated. The crude product was extracted in MeOH, trimethylamine N-oxide (2.0 g, 26.6 mmol) was added, and the solution was brought to reflux for 2 hours. The reaction mixture was cooled, concentrated and the crude residue purified by column chromatography (SiO₂, methylene chloride) to afford 5-bromo-3,3-dimethyl-2,3-dihydro-1H-indole (0.074 g, 87%) as a yellow oil: ¹H NMR (CDCl₃) δ 1.29 (s, 6H), 3.30 (s, 2H), 3.5 (br s, 1H), 6.49 (d, *J* = 8.8 Hz, 1H), 7.08-7.12 (m, 2H); ¹³C-NMR (CDCl₃) δ 27.69 (q), 42.21 (s), 62.01 (d), 110.38 (s), 111.09, 125.46, 130.09 (d), 141.03, 149.52 (s); MS (EI*) m*/*z* 225, 227 (M)⁺.

Using the standard coupling conditions given in Example 1, the title compound was prepared from 5-bromo-3,3-dimethyl-2,3-dihydro-1H-indole (0.25 g, 1.1 mmol), tetrakis(triphenylphosphine) palladium (0.08 g, 0.07 mmol) in dimethoxyethane (5 mL) with 3-nitrophenylboronic acid (0.22 g, 1.3 mmol) and sodium carbonate (0.35 g, 3.3 mmol) in 5 mL water. The title compound (0.17 g, 60%) was obtained as a brown semi-solid: ¹H NMR (CDCl₃) δ 1.38 (s, 3H), 3.40 (s, 2H), 6.72 (d, *J* = 8.0 Hz, 1H), 7.29-7.34 (m, 2H), 7.54 (dd, *J =* 8.0, 8.0 Hz, 1H), 7.86 (d, *J* = 7.8 Hz, 1H), 8.09 (dd, *J =* 8.0,1.5 Hz, 1H), 8.40 (dd, *J* = 2.0, 2.0 Hz, 1H); ¹³C-NMR (CDCl₃) δ 27.89 (q), 41.93 (s), 62.05 (d), 109.78, 120.87, 121.02, 121.23, 126.87 (d), 129.19 (s), 129.69, 132.52 (d), 139.64, 143.75, 148.94, 151.17 (s); MS (EI*) m*/*z* 268 (M)⁺.

### EXAMPLE 9

### 5'-(3-Chlorophenyl)-1',2'-dihydrospiro[cyclohexane-1,3'-[3H]indole]

### Spiro[cyclohexane-1,3'-[3H]indol]-2'-(1'H) one

A solution of the oxindole (25g, 190 mmol) in 800 mL of anhydrous THF was cooled to -20°C, *n*-butyllithium (2.5M in hexanes, 152 mL, 0.38 mol) slowly added, followed by the addition of N,N,N',N'-tetramethylenediamine (51 mL, 0.38 mol,). After 15 minutes, 1,5-diiodopentane (174g, 0.54 mol) was added slowly and the mixture was allowed to warm to room temperature. After stirring for 16 hours saturated aqueous ammonium chloride solution (1L) and ethylacetate (1L) were added. After 15 minutes, the layers were separated and the aqueous phase was extracted with ethyl acetate. The combined organic layers were extracted with hydrochloric acid (1N, 500 mL), then washed with brine, dried (MgSO₄), and concentrated to obtain an oil. The oil was triturated with hexane (200 mL) and benzene (20 mL). The precipitate was collected and dried *in vacuo* to provide Spiro [cyclohexane-1,3'-[3H]indol]-2'-(1'H) one (26.3g, 69.6%) as colorless crystals: mp 110-114°; ¹H NMR (DMSO-d₆) δ 1.67 (m, 10H), 6.84 (d, 1H, *J* = 8 Hz), 6.94 (t, 1H, *J* = 8 Hz), 7.17 (t, 1H, *J* = 8 Hz), 7.44 (d, 1H, *J* = 8 Hz), 10.3 (s, 1H).

To a solution of the above oxindole (17.6 g, 90.0 mmol) in acetic acid (300 mL) was added sodium acetate (8.0 g, 100.0 mmol) and bromine (14.6g, 91.0 mmol) with stirring. After 30 minutes at room temperature, the reaction mixture was partitioned between water and ethylacetate. The aqueous phase was extracted with ethylacetate. The combined organic layers were washed with water, dried (MgSO₄) and evaporated. The residue was triturated with hexane. The precipitate was collected, and dried *in vacuo* to provide 5-Bromo-spiro[cyclohexane-1,3'-[3H]indol]-2'(1'H)-one (16.5g, 67%) as off-white crystals: m.p. 196-199°; ¹H NMR (DMSO-d₆) δ 1.62 (m, 10H), 6.8 (d, 1H, *J =* 6.8 Hz), 7.36 (d, 1H, *J =* 8.2,1.8 Hz), 7.58 (dd, 1H, *J* = 8.2, 1.8 Hz), 10.44 (s, 1H).

Using the standard coupling conditions given in Example 1, the title compound was prepared from the above bromo-oxindole (0.32 g, 1.14 mmol) tetrakis(triphenylphosphine) palladium (0.08g, 0.07 mmol) and 3-chlorophenylboronic acid (0.21 g, 1.37 mmol), and sodium carbonate (0.36 g, 3.4 mmol) in water (3 mL). 5-(3-chlorophenyl)-spiro[cyclohexane-1,3-[3H]indol]-2(1H)-one (0.28 g, 80%) was obtained as a yellow solid: m.p. 164-165 °; ¹H NMR (CDCl₃) δ 1.60-1.78 (m, 6H), 1.81-1.99 (m, 4H), 7.04 (d, *J*= 8.1 Hz, 1H), 7.22-7.47 (m, 4H), 7.53 (s, 1H), 7.61 (s, 1H), 9.28 (br s, 1H); ¹³C-NMR (CDCl₃) δ 20.17, 24.12, 31.92 (t), 47.22 (s), 109.21, 121.94, 124.06, 125.50, 125.79, 125.97, 126.38, 128.96 (d), 132.88, 133.59, 135.60, 139.14, 142.17, 182.89 (s); MS (EI*) m*/*z* 310, 312 (M-H)⁺.

To a solution of 5-(3-chlorophenyl)-spiro[cyclohexane-1,3-[3H]indol]-2(1H)-one (0.42 g, 1.4 mmol) in 20 mL THF at 0 °C is added borane-dimethylsulfide complex, (2M in THF, 14 mL, 28 mmol). The reaction mixture was warmed to room temperature then brought to reflux for 4 hours The mixture was cooled to room temperature and poured into H₂O with CH₂Cl₂ and washed with 5% NaHCO₃. The organic layer was washed with brine, dried over Na₂SO₄ and concentrated. The crude product was extracted in MeOH and trimethylamine N-oxide (1.0 g, 9.0 mmol) was added. The solution was brought to reflux for 2 hours, the mixture was cooled, concentrated and the crude residue purified by column chromatography (SiO₂, methylene chloride) to afford the title compound (0.25 g, 63%) as a yellow oil: ¹H NMR (CDCl₃) δ 1.3-1.55 (m, 3H), 1.6-1.85 (m, 7H), 3.1 (br s, 1H), 3.5 (s, 2H), 6.6 (d, J = 8.0 Hz, 1H), 7.18-7.32 (m, 4H), 7.4 (dd, J = 7.7, 1.5 Hz, 1H), 7.5 (s, 1H); ¹³C-NMR (CDCl₃) δ 23.65, 26.24, 37.00 (t), 46.58 (s), 57.49 (t), 109.95, 121.82, 125.07, 126.36, 126.98, 127.08, 130.29 (d), 130.64, 134.91, 139.65, 144.29, 151.09 (s); MS (EI*) m*/*z* 298, 300 (M+H)⁺.

### EXAMPLE 10

### 3-(2',3',3'-trimethyl-2',3-dihydro-1H-indol-5'-yl)benzonitrile

To a solution of 5-bromo-2,3,3-trimethyl-2,3-dihydro-1H-indole (2.03 g, 8.45 mmol) in dimethoxyethane (50 mL), under nitrogen was added tetraltis(triphenylphosphine)palladium (0.47 g, 0.4 mmol). After 20 minutes at room temperature, 3-formylphenylboronic acid (2.36 g, 16.4 mmol) and potassium carbonate (6.80 g, 55 mmol) in water (25 mL) was added, and the mixture was heated under reflux. After 2 hours, the mixture was cooled, poured into brine and extracted with EtOAc (x 2). The combined organic extracts were washed with brine, dried (MgSO₄) and evaporated. The residue was then subjected to column chromatography (SiO₂, EtOAc:hexane, 1:8) to afford 3-(2',3,3'-trimethyl-2',3-dihydro-1H-indol-5'-yl) benzaldehyde (0.96 g, 3.62 mmol, 43 %) as a slightly impure solid that was used without further purification: ¹H NMR (CDCl₃) δ 1.11 (s, 3H), 1.22 (d, 3H, *J* = 6.5 Hz), 1.35 (s, 3H), 3.59 (dd, 1H, *J* = 13, 7 Hz), 6.69 (d, 1H, *J* = 1 Hz), 7.25 (s, 1H), 7.32 (s, 1H), 7.55 (t, 1H, *J* = 7.6 Hz), 7.80 (d, 1H, *J* = 1 Hz), 7.82 (d, 1H, *J* = 1 Hz), 8.05 (s, 1H), 10.07 (s, 1H).

To a solution of the above compound (0.96 g, 3.62 mmol) in MeCN/H₂O (20 mL, 95:5) was added hydroxylamine hydrochloride (0.82 g, 7.25 mmol). After 1 hour, the mixture was poured into a saturated sodium hydrogen carbonate solution and extracted with EtOAc (x2). The combined organic extracts were washed with water, dried (MgSO₄) and evaporated. The residue was then dissolved in dichloromethane (20 mL) and treated with thionyl chloride (0.53 mL, 7.25 mmol). After 16 hours, the mixture was quenched with saturated sodium hydrogen carbonate solution and concentrated, partitioned between water and EtOAc, and re-extracted with EtOAc. The combined organic extracts were washed with brine, dried (MgSO₄) and evaporated. Purification by column chromatography (EtOAc: hexane, 1:8) afforded a yellow oil (0.31 g) which was dissolved in methanol (5 mL) and treated with ethereal hydrogen chloride (1M, 1.3 mL, 1.3 mmol) and evaporated. Recrystallization from MeOH/Et₂O then afforded the title compound (0.20 g, 0.60 mmol, 18 %): mp >235 °C (decomp), ¹H NMR (CDCl₃) δ 1.57 (s, 3H), 1.36 (d, 3H, *J* = 6.7 Hz), 1.40 (s, 3H),3.72-3.76 (m, 1H), 7.30 (d, 1H, J = 8 Hz), 7.67 (t, 2H, J = 7 Hz), 7.79 - 7.84 (m, 2H), 8.03 (d, 1H, J = 8 Hz), (8.2, s, 1H); MS (EI) *m*/*z* 262 (M)⁺.

### EXAMPLE 11 - Pharmacology

The biological activity for the compounds of the current invention was evaluated in the in-vitro and in-vivo assays described below. In-vitro potencies lie in the range 0.01 nM - 10,000 nM, and in-vivo potencies in the range 1 µg/kg to 100 mg/kg.

### A. In-vitro biology

The in-vitro biology is determined by (1) competitive radioligand Binding: using the A-form of the human progesterone receptor with progesterone as the radioligand; (2) co-transfection assay, which provides functional activity expressed as agonist EC50 and Antagonist IC50 values; and (3) a T47D cell proliferation, which is a further functional assay which also provides agonist and antagonist data.
1. hPR Binding assay - This assay is carried out in accordance with: Pathirana, C.; Stein, R.B.; Berger, T.S.; Fenical, W.; Ianiro, T.; Mais, D.E.; Torres, A.; Glodman, M.E., Nonsteroidal human progesterone receptor modulators from the marine alga cymoplia barbata, J. Steroid Biochem. Mol. Biol., 1992, 41, 733-738.
2. PRE-luciferase assay in CV-1 cells
The object of this assay is to determine a compound's progestational or antiprogestational potency based on its effect on PRE-luciferase reporter activity in CV-1 cells co-transfected with human PR and PRE-luciferase plasmids. The materials methods used in the assay are as follows.
a. Medium: The growth medium was as follows: DMEM (BioWhittaker) containing 10% (v/v) fetal bovine serum (heat inactivated), 0.1 mM MEM non-essential amino acids, 100U/ml penicillin, 100mg/ml streptomycin, and 2 mM GlutaMax (GIBCO, BRL). The experimental medium was as follows: DMEM (BioWhittaker), phenol red-free, containing 10% (v/v) charcoal-stripped fetal bovine serum (heat-inactivated), 0.1 mM MEM non-essential amino acids, 100U/ml penicillin, 100mg/ml streptomycin, and 2 mM GlutaMax (GIBCO, BRL).
b. Cell culture, transfection, treatment, and luciferase assay
Stock CV-1 cells are maintained in growth medium. Co-transfection is done using 1.2x10⁷ cells, 5 mg pLEM plasmid with hPR-B inserted at Sph1 and BamH1 sites, 10 mg pGL3 plasmid with two PREs upstream of the luciferase sequence, and 50 mg sonicated calf thymus DNA as carrier DNA in 250 ml. Electroporation is carried out at 260 V and 1,000 mF in a Biorad Gene Pulser II. After electroporation, cells are resuspended in growth medium and plated in 96-well plate at 40,000 cells/well in 200 µl. Following overnight incubation, the medium is changed to experimental medium. Cells are then treated with reference or test compounds in experimental medium. Compounds are tested for antiprogestational activity in the presence of 3 nM progesterone. Twenty-four hr. after treatment, the medium is discarded, cells are washed three times with D-PBS (GIBCO, BRL). Fifty µl of cell lysis buffer (Promega, Madison, WI) is added to each well and the plates are shaken for 15 min in a Titer Plate Shaker (Lab Line Instrument, Inc.). Luciferase activity is measured using luciferase reagents from Promega.
c. Analysis of Results:
Each treatment consists of at least 4 replicates. Log transformed data are used for analysis of variance and nonlinear dose response curve fitting for both agonist and antagonist modes. Huber weighting is used to downweight the effects of outliers. EC₅₀ or IC₅₀ values are calculated from the retransformed values. JMP software (SAS Institute, Inc.) is used for both one-way analysis of variance and non-linear response analyses.
d. Reference Compounds:
Progesterone and trimegestone are reference progestins and RU486 is the reference antiprogestin. All reference compounds are run in full dose-response curves and the EC₅₀ or IC₅₀ values are calculated.

**Table 1.**

| **Estimated EC**_{**50**}**, standard error (SE), and 95% confidence intervals** **(CI) for reference progestins from three individual studies** | | | | | |
|---|---|---|---|---|---|
| | EC50 | | | 95% CI | |
| Compound | Exp. | (nM) | SE | lower | upper |
| Progesterone | 1 | 0.616 | 0.026 | 0.509 | 0.746 |
| | 2 | 0.402 | 0.019 | 0.323 | 0.501 |
| | 3 | 0.486 | 0.028 | 0.371 | 0.637 |
| | | | | | |
| Trimegestone | 1 | 0.0075 | 0.0002 | 0.0066 | 0.0085 |
| | 2 | 0.0081 | 0.0003 | 0.0070 | 0.0094 |
| | 3 | 0.0067 | 0.0003 | 0.0055 | 0.0082 |

**Table 2.**

| **Estimated IC**_{**50**}**, standard error (SE), and 95% confident interval (CI)** **for the antiprogestin, RU486 from three individual studies** | | | | | |
|---|---|---|---|---|---|
| | IC 50 | | | 95% CI | |
| Compound | Exp. | (nM) | SE | lower | upper |
| RU486 | 1 | 0.028 | 0.002 | 0.019 | 0.042 |
| | 2 | 0.037 | 0.002 | 0.029 | 0.048 |
| | 3 | 0.019 | 0.001 | 0.013 | 0.027 |

Progestational activity: Compounds that increase PRE-luciferase activity significantly (p<0.05) compared to vehicle control are considered active.
Antiprogestational activity: Compounds that decrease 3 nM progesterone induced PRE-luciferase activity significantly (p<0.05)
EC₅₀: Concentration of a compound that gives half-maximal increase PRE-luciferase activity (default-nM) with SE.
IC₅₀: Concentration of a compound that gives half-maximal decrease in 3 nM progesterone induced PRE-luciferase activity (default-nM) with SE.
3. T47D cell proliferation assay
The objective of this assay is the determination of progestational and antiprogestational potency by using a cell proliferation assay in T47D cells. A compound's effect on DNA synthesis in T47D cells is measured. The materials and methods used in this assay are as follows.
a. Growth medium: DMEM:F12 (1:1) (GIBCO, BRL) supplemented with 10% (v/v) fetal bovine serum (not heat-inactivated), 100U/ml penicillin, 100mg/ml streptomycin, and 2 mM GlutaMax (GIBCO, BRL).
b. Treatment medium: Minimum Essential Medium (MEM) (#51200-038GIBCO, BRL) phenol red-free supplemented with 0.5% charcoal stripped fetal bovine serum, 100U/ml penicillin, 200 mg/ml streptomycin, and 2 mM GlutaMax (GIBCO, BRL).
c. Cell culture
Stock T47 D cells are maintained in growth medium. For BrdU incorporation assay, cells are plated in 96-well plates (Falcon, Becton Dickinson Labware) at 10,000 cells/well in growth medium. After overnight incubation, the medium is changed to treatment medium and cells are cultured for an additional 24 hr before treatment. Stock compounds are dissolved in appropriate vehicle (100% ethanol or 50% ethanol/50% DMSO), subsequently diluted in treatment medium and added to the cells. Progestin and antiprogestin reference compounds are run in full dose-response curves. The final concentration of vehicle is 0.1%. In control wells, cells receive vehicle only. Antiprogestins are tested in the presence of 0.03 nM trimegestone, the reference progestin agonist. Twenty-four hours after treatment, the medium is discarded and cells are labeled with 10 mM BrdU (Amersham Life Science, Arlington Heights, IL) in treatment medium for 4 hr.
d. Cell Proliferation Assay
At the end of BrdU labeling, the medium is removed and BrdU incorporation is measured using a cell proliferation ELISA kit (#RPN 250, Amersham Life Science) according to manufacturer's instructions. Briefly, cells are fixed in an ethanol containing fixative for 30 min, followed by incubation in a blocking buffer for 30 min to reduce background. Peroxidase-labeled anti-BrdU antibody is added to the wells and incubated for 60 min. The cells are rinsed three times with PBS and incubated with 3,3'5,5'-tetramethylbenzidine (TMB) substrate for 10-20 min depending upon the potency of tested compounds. Then 25 µl of 1 M sulfuric acid is added to each well to stop color reaction and optical density is read in a plate reader at 450 nm within 5 min.
e. Analysis of Results:
Square root-transformed data are used for analysis of variance and nonlinear dose response curve fitting for both agonist and antagonist modes. Huber weighting is used to downweight the effects of outliers. EC₅₀ or IC₅₀ values are calculated from the retransformed values. JMP software (SAS Institute, Inc.) is used for both one-way analysis of variance and non-linear dose response analyses in both single dose and dose response studies.
f. Reference Compounds:
Trimegestone and medroxyprogesterone acetate (MPA) are reference progestins and RU486 is the reference antiprogestin. All reference compounds are run in full dose-response curves and the EC₅₀ or IC₅₀ values are calculated.

**Table 3.**

| **Estimated EC**_{**50**}**, standard error (SE), and 95% confidence intervals** **(CI) for individual studies** | | | | | |
|---|---|---|---|---|---|
| | EC₅₀ | | | 95% CI | |
| Compound | Exp | (nM) | SE | lower | upper |
| Trimegestone | 1 | 0.017 | 0.003 | 0.007 | 0.040 |
| | 2 | 0.014 | 0.001 | 0.011 | 0.017 |
| | 3 | 0.019 | 0.001 | 0.016 | 0.024 |
| | | | | | |
| MPA | 1 | 0.019 | 0.001 | 0.013 | 0.027 |
| | 2 | 0.017 | 0.001 | 0.011 | 0.024 |

**Table 4.**

| **Estimated IC**_{**50**}**, standard error, and 95% confident interval for the** **antiprogestin, RU486** | | | | | |
|---|---|---|---|---|---|
| | IC₅₀ | | | 95% CI | |
| Compound | Exp | (nM) | SE | lower | upper |
| RU486 | 1 | 0.011 | 0.001 | 0.008 | 0.014 |
| | 2 | 0.016 | 0.001 | 0.014 | 0.020 |
| | 3 | 0.018 | 0.001 | 0.014 | 0.022 |

EC₅₀: Concentration of a compound that gives half-maximal increase in BrdU incorporation with SE; IC₅₀: Concentration of a compound that gives half-maximal decrease in 0.1 trimegestone induced BrdU incorporation with SE.

## Claims

1. A compound of the formula: 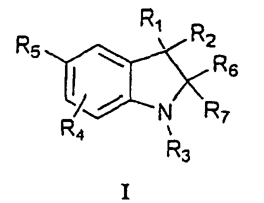 wherein:
R₁ and R₂ are selected independently from the group consisting of H, C₁ to C₈ alkyl, substituted C₁ to C₈ alkyl, OH, O (C₁ to C₈ alkyl), O(substituted C₁ to C₈ alkyl), O(acetyl), aryl, substituted aryl, heteroaryl, substituted heteroaryl, C₁ to C₈ alkylaryl, C₁ to C₈ alkylheteroaryl, 1-propynyl, and 3-propynyl;
or R₁ and R₂ are joined to form a ring comprising -CH₂(CH₂)ₙCH₂-, -CH₂CH₂C(CH₃)₂CH₂CH₂-, -O(CH₂)ₘCH₂-; -O(CH₂)ₚO-, -CH₂CH₂OCH₂CH₂-; -CH₂CH₂N(C₁ to C₈ alkyl)CH₂CH₂-, or -CH₂CH₂NHCH₂CH₂-;
n is an integer from 0 to 5;
m is an integer from 1 to 4;
p is an integer from 1 to 4;
or R₁ and R₂ together comprise a double bond to C(CH₃)₂, C(C₃ to C₆ cycloalkyl), O, or C(cycloether);
wherein said cycloether is selected from the group consisting of tetrahydrofurartyl and hexahydropyranyl;
R₃ is H, OH, NH₂, C₁ to C₆ alkyl, substituted C₁ to C₆ alkyl, C₃ to C₆ alkenyl, C₂ to C₈ alkynyl, substituted C₂ to C₈ alkynyl, or COR^{A};
R^{A} is H, C₁ to C₃ alkyl, substituted C₁ to C₃ alkyl, C₁ to C₃ alkyloxy, substituted C₁ to C₃ alkyloxy, amino C₁ to C₃ alkyl, or amino substituted C₁ to C₃ alkyl;
R₄ is H, halogen, CN, NH₂, C₁ to C₆ alkyl, substituted C₁ to C₆ alkyl, C₁ to C₆ alkyloxy, substituted C₁ to C₆ alkyloxy, amino C₁ to C₆ alkyl, or amino substituted C₁ to C₆ alkyl;
R₅ is selected from the group consisting of a), b) and c):
a) a substituted benzene ring of the formula: 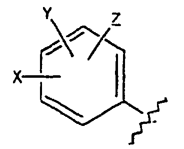 X is selected from the group consisting of halogen, OH, CN, C₁ to C₃ alkyl, substituted C₁ to C₃ alkyl, C₁ to C₃ alkyloxy, substituted C₁ to C₃ alkyloxy, thio C₁ to C₃ alkyl, thio substituted C₁ to C₃ alkyl, S(O) C₁ to C₈ alkyl, S(O)₂ C₁ to C₈ alkyl, amino C₁ to C₃ alkyl, substituted amino C₁ to C₃ alkyl, NO₂, C₁ to C₃ perfluoroalkyl, 5 or 6 membered heterocyclic ring containing in its backbone 1 to 3 heteroatoms, COR^{B}, OCOR^{B}, and NR^{C}COR^{B};
R^{B} is H, C₁ to C₃ alkyl, substituted C₁ to C₃ alkyl, aryl, substituted aryl, C₁ to C₃ alkyloxy, substituted C₁ to C₃ alkyloxy, amino C₁ to C₃ alkyl, or amino substituted C₁ to C₃ alkyl;
R^{C} is H, C₁ to C₃ alkyl, or substituted C₁ to C₃ alkyl;
Y and Z are independently selected from the group consisting of H, halogen, CN, NO₂, C₁ to C₃ alkyloxy, C₁ to C₃ alkyl, and thio C₁ to C₃ alkyl;
b) a five or six membered ring having in its backbone 1, 2, or 3 heteroatoms selected from the group consisting of O, S, SO, SO₂ and NR₈ and containing one or two independent substituents selected from the group consisting of H, halogen, CN, NO₂, C₁ to C₃ alkyl, C₁ to C₃ alkyloxy, amino C₁ to C₃ alkyl, COR^{D}, and NR^{E}COR^{D};
R^{D} is H, C₁ to C₃ alkyl, substituted C₁ to C₃ alkyl, aryl, substituted aryl, C₁ to C₃ alkyloxy, substituted C₁ to C₃ alkyloxy, amino C₁ to C₃ alkyl, or amino substituted C₁ to C₃ alkyl;
R^{E} is H, C₁ to C₃ alkyl, or substituted C₁ to C₃ alkyl;
R₈ is H or C₁ to C₃ alkyl; and
c) an indol-4-yl, indol-7-yl or benzo-2-thiophene moiety, the moiety being optionally substituted by from 1 to 3 substituents selected from the group consisting of halogen, C₁ to C₆ alkyl, CN, NO₂, C₁ to C₆ alkyloxy, and CF₃;
R₆ and R₇ are independently selected from the group consisting of H, methyl, ethyl, propyl, butyl, iso-propyl, iso-butyl, cyclohexyl, aryl, substituted aryl, heteroaryl, and substituted heteroaryl;
or pharmaceutically acceptable salt thereof;
in which compounds the term "substituted" in the expression "substituted alkyl" denotes the presence of one or more substituents selected from the group consisting of halogen, CN, OH, NO₂, amino, aryl, heterocyclic, substituted aryl, substituted heterocyclic, C₁ to C₈ alkyloxy, aryloxy, substituted C₁ to C₈ alkyloxy, C₁ to C₈ alkylcarbonyl, C₁ to C₈ alkylcarboxy, C₁ to C₈ alkylamino and arylthio;
the term "substituted" in the expression "substituted aryl" denotes the presence of 1 to 4 substituents selected from the group consisting of halogen, CN, OH, NO₂, amino, C₁ to C₈ alkyl, cycloalkyl, C₂ to C₈ alkenyl, C₂ to C₈ alkynyl, C₁ to C₈ alkoxy, aryloxy, substituted C₁ to C₈ alkyloxy, C₁ to C₈ alkylcarbonyl, C₁ to C₈ alkylcarboxy, C₁ to C₈ alkylamino and arylthio;
the term "hetero aryl" denotes aryl which comprises a 4 - to 7- membered monocyclic heterocyclic ring or a multicyclic ring which consists of carbon atoms and from 1 to 4 heteroatoms selected from the group consisting of N, O and S atoms or a multicyclic ring system in which any of the aforesaid heterocyclic rings is fused to an aryl ring;
the term "substituted" in the expression "substituted heteroaryl" denotes the presence of 1 to 4 substituents selected from the group consisting of halogen, CN, OH, NO₂, amino, C₁ to C₈ alkyl, substituted C₁ to C₈ alkyl, cycloalkyl, C₂ to C₈ alkenyl, substituted C₂ to C₈ alkenyl, C₂ to C₈ alkynyl, substituted C₂ to C₈ alkynyl, C₁ to C₈ allcyloxy, aryloxy, substituted C₁ to C₈ alkyloxy, C₁ to C₈ alkylcarbonyl, C₁ to C₈ alkylcarboxy, C₁ to C₈ alkylamino and arylthio;
the term "substituted" in the expressions "substituted alkynyl" and "substituted alkenyl" has the same meanings as in the expression "substituted alkyl";
the term "heterocyclic" denotes a 4 - to 7 - membered monocyclic heterocyclic ring or a multicyclic ring which consists of carbon atoms and from 1 to 4 heteroatoms selected from the group consisting of N, O and S atoms or a multicyclic ring system in which any of the aforesaid heterocyclic rings is fused to an aryl ring; and
the term "substituted" in the expression "substituted heterocyclic" has the same meanings as in the expression "substituted heteroaryl".

2. A compound of the formula: 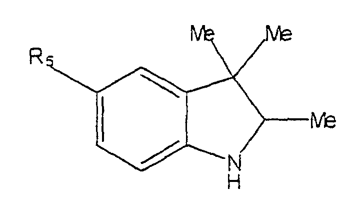 wherein:
R₅ is (i) or (ii):
(i) a substituted benzene ring of the formula: 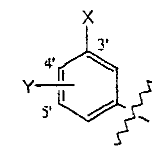
X is selected from the group consisting of halogen, CN, C₁ to C₃ alkyloxy, C₁ to C₃ alkyl, NO₂, C₁ to C₃ perfluoroalkyl 5-membered heterocyclic ring containing in its backbone 1 to 3 heteroatoms, and thio C₁ to C₃ alkoxy;
Y is on the 4' or 5' position and is selected from the group consisting of H, halogen, CN, NO₂, C₁ to C₃ alkyloxy, C₁ to C₄ alkyl, and thio C₁ to C₃ alkyl;
(ii) a 5-membered heterocyclic ring of the formula: 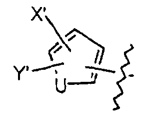 wherein:
U is O, S, or NR₈;
R₈ is H, C₁ to C₃ alkyl, or C₁ to C₄ CO₂alkyl;
X' is selected from the group consisting of halogen, CN, NO₂, C₁ to C₃ alkyl, and C₁ to C₃ alkyloxy;
Y' is selected from the group consisting ofH, halogen, CN, NO₂, and C₁ to C₄ alkyl;
wherein said halogen is F;
or pharmaceutically acceptable salt thereof.

3. A compound which is 4a-Methyl-6-(3-nitro-phenyl)-2,3,4,4a,9,9a-hexahydro-1H-carbazole or a pharmaceutically acceptable salt thereof.

4. The compound according to Claim 2 of the formula: 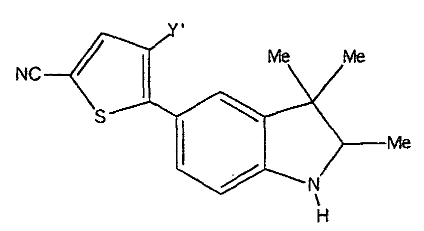 wherein Y' is H or C₁ to C₄ alkyl.

5. The compound according to Claim 1 or 2 of the formula: 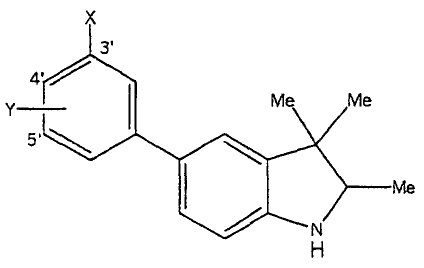 wherein:
X is selected from the group consisting of halogen, CN, C₁ to C₃ alkyloxy, C₁ to C₃ alkyl, NO₂, C₁ to C₃ perfluoroalkyl, 5-membered heterocyclic ring containing in its backbone 1 to 3 heteroatoms, and thio C₁ to C₃ alkoxy;
Y is on the 4' or 5' position and is selected from the group consisting of H, halogen, CN, NO₂, C₁ to C₃ alkyloxy, C₁ to C₃ alkyl, and thio C₁ to C₃ alkyl.

6. The compound according to Claim 2 or 3, wherein X is CN or NO₂ and Y is 5'-fluoro.

7. The compound according to Claim 1 or 2 of the formula: 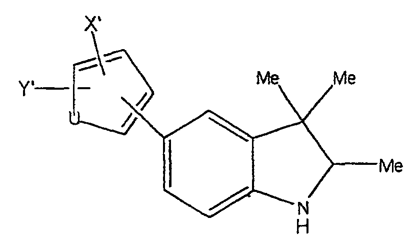 wherein:
U is O, S, or NR₈ ;
R₈ is H or C₁ to C₃ alkyl;
X' is selected from the group consisting of halogen, CN, NO₂, C₁ to C₃ alkyl, and C₁ to C₃ alkyloxy;
Y' is selected from the group consisting of H, halogen, CN, NO₂, and C₁ to C₃ alkyl;
wherein said halogen is F.

8. The compound according to any of claims 1, 2, 4 or 7 of the formula: 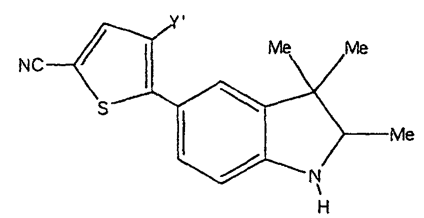 wherein Y' is H or C₁ to C₃ alkyl.

9. The compound according to any of claims 1, 2 or 7 of the formula:

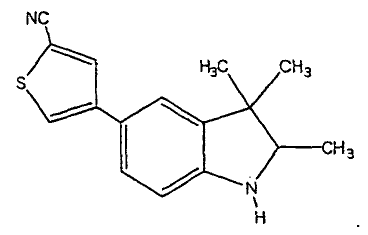

10. The compound according to Claim 1 of the formula: 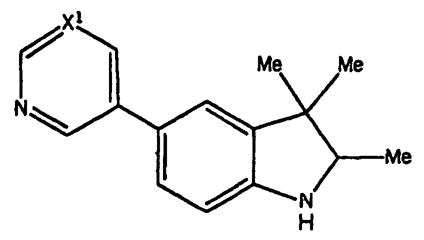 wherein:
X¹ is N or CX²;
X² is halogen, CN or NO₂.

11. The compound according to Claim 1, which is 2,3,3-Trimethyl-5-(3-nitro-phenyl)-2,3-dihydro-1H-indole, 2,3,3-Trimethyl-5-(3-nitro-phenyl)-2,3-dihydro-1H-indole Hydrochloride, (2-R)-2,3,3-Trimethyl-5-(3-nitro-phenyl)-2,3-dihydro-1H-indole, (2-S)-2,3,3-Trimethyl-5-(3-nitro-phenyl)-2,3-dihydro-1H-indole, 2,3,3-Diethyl-2-methyl-5-(3-nitro-phenyl)-2,3-dihydro-1H-indole, 1,2-Dihydro-2-methyl-5-(3-nitro-phenyl)spiro[cyclohexane-1,3-[3H]indole], 3,3-Dimethyl-5-(3-nitro-phenyl)-2,3-dihydro-1H-indole, 5'-(3-Chlorophenyl)-1',2'-dihydrospiro[cyclohexane-1,3'-[3H]indole], and 3-(2',3',3'-trimethyl-2',3-dihydro-1H-indol -5'-yl) benzonitrile, or a pharmaceutically acceptable salt thereof

12. A pharmaceutical composition comprising a pharmaceutically effective amount of a compound according to any of claims 1 to 11 and a pharmaceutically acceptable carrier or excipient.

13. Use of a compound according to any of claims 1 to 11, or said acceptable salt thereof, in preparing a medicament.

14. Use of the compound according to any of claims 1 to 11, or said acceptable salt, in preparing a medicament useful for administration to a mammalian subject as a contraceptive.

15. Use of the compound according to any of claims 1 to 11, or said acceptable salt, in preparing a medicament useful for administration to a mammalian subject for treating or preventing benign or malignant neoplastic disease.

16. The use according to Claim 15, wherein the benign or malignant neoplastic disease is selected from the group consisting of uterine myometrial fibroids, endometriosis, benign prostatic hypertrophy, carcinomas and. adenocarcinomas of the endometrium, ovary, breast, colon, prostate, pituitary, meningioma and other hormone-dependent tumors.

17. Use of the compound according to any of claims 1 to 11, or said acceptable salt, in preparing a medicament for administration to a mammalian subject for hormone replacement therapy.

## Patentansprüche

1. Verbindungen der Formel 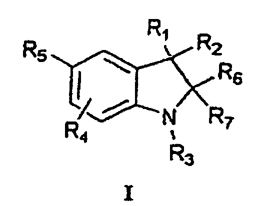 in welcher:
R₁ und R₂ unabhängig voneinander aus der aus H, C₁-C₈-Alkyl, substituiertem C₁-C₈-Alkyl, OH, O-(C₁-C₈-Alkyl), O-(substituiertes C₁-C₈-Alkyl), O-(Acetyl), Aryl, substituiertem Aryl, Heteroaryl, substituiertem Heteroaryl, C₁-C₈-Alkylary, C₁-C₈-Alkylheteroaryl, 1-Propinyl und 3-Propinyl bestehenden Gruppe ausgewählt sind;
oder R₁ und R₂ miteinander verbunden sind und einen Ring bilden, der -CH₂(CH₂)ₙCH₂-, -CH₂CH₂C(CH₃)₂CH₂CH₂-, -O(CH₂)ₘCH₂-, -O(CH₂)ₚO-, -CH₂CH₂OCH₂CH₂-, -CH₂CH₂N(C₁-C₈-Alkyl)CH₂CH₂-, oder -CH₂CH₂NHCH₂CH₂-, enthält;
n für eine ganze Zahl von 0 bis 5 steht;
m für eine ganze Zahl von 1 bis 4 steht;
p für eine ganze Zahl von 1 bis 4 steht;
oder R₁ und R₂ zusammen für eine Doppelbindung an C(CH₃)₂, C(C₃-C₆-Cycloalkyl), O oder C-(Cycloether) stehen;
wobei der Cycloether aus der aus Tetrahydrofuranyl und Hexahydropyranyl bestehenden Gruppe ausgewählt ist;
R₃ für H, OH, NH₂, C₁-C₆-Alkyl, substituiertes C₁-C₆-Alkyl, C₃-C₆-Alkenyl, C₂-C₈-Alkinyl, substituiertes C₂-C₈-Alkinyl oder COR^{A} steht;
R^{A} für H, C₁-C₃-Alkyl, substituiertes C₁-C₃-Alkyl, C₁-C₃-Alkyloxy, substituiertes C₁-C₃-Alkyloxy, Amino-C₁-C₃-Alkyl oder Amino-(substituiertes C₁-C₃-Alkyl) steht;
R₄ für H, Halogen, CN, NH₂, C₁-C₆-Alkyl, substituiertes C₁-C₆-Alkyl, C₁-C₆-Alkyloxy, substituiertes C₁-C₆-Alkyloxy, Amino-C₁-C₆-Alkyl oder Amino-(substituiertes C₁-C₆-Alkyl) steht;
R₅ aus der aus a), b) und c) bestehenden Gruppe ausgewählt ist:
a) ein substituierter Benzolring der Formel: 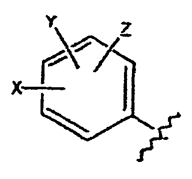
X ist aus der aus Halogen, OH, CN, C₁-C₃-Alkyl, substituiertem C₁-C₃-Alkyl, C₁-C₃-Alkyloxy, substituiertem C₁-C₃-Alkyloxy, Thio-C₁-C₃-Alkyl, Thio-(substituiertes C₁-C₃-Alkyl), S(O)-C₁-C₈-Alkyl, S(O)₂-C₁-C₈-Alkyl, Amino-C₁-C₃-Alkyl, Amino-(substituiertes C₁-C₃-Alkyl), NO₂, C₁-C₃-Perfluoralkyl, einem 5- oder 6gliedrigen heterocyclischen Ring mit 1 bis 3 Heteroatomen im Gerüst, COR^{B}, OCOR^{B} und NR^{C}COR^{B} bestehenden Gruppe ausgewählt;
R^{B} steht für H, C₁-C₃-Alkyl, substituiertes C₁-C₃-Alkyl, Ary, substituiertes Aryl, C₁-C₃-Alkyloxy, substituiertes C₁-C₃-Alkyloxy, Amino-C₁-C₃-Alkyl oder Amino-(substituiertes C₁-C₃-Alkyl);
R^{C} steht für H, C₁-C₃-Alkyl oder substituiertes C₁-C₃-Alkyl;
Y und Z sind unabhängig voneinander aus der aus H, Halogen, CN, NO₂, C₁-C₃-Alkyloxy, C₁-C₃-Alkyl und Thio-C₁-C₃-Alkyl bestehenden Gruppe ausgewählt;
b) ein 5- oder 6gliedriger Ring mit 1, 2 oder 3 aus der aus O, S, SO, SO₂ und NR₈ bestehenden Gruppe ausgewählten Heteroatomen im Gerüst und einem oder zwei voneinander unabhängigen Substituenten, ausgewählt aus der aus H, Halogen, CN, NO₂, C₁-C₃-Alkyl, C₁-C₃-Alkyloxy, Amino-C₁-C₃-alkyl, COR^{D} und NR^{E}COR^{D} bestehenden Gruppe;
R^{D} steht für H, C₁-C₃-Alkyl, substituiertes C₁-C₃-Alkyl, Aryl, substituiertes Aryl, C₁-C₃-Alkyloxy, substituiertes C₁-C₃-Alkyloxy, Amino-C₁-C₃-alkyl oder Amino-(substituiertes C₁-C₃-Alkyl);
R^{E} steht für H, C₁-C₃-Alkyl oder substituiertes C₁-C₃-Alkyl;
R₈ steht für H oder C₁-C₃-Alkyl; und
c) eine Indol-4-yl, Indol-7-yl oder Benzo-2-thiopheneinheit, wobei die Einheit gegebenenfalls durch 1 bis 3 Substituenten ausgewählt aus der aus Halogen, C₁-C₆-Alkyl, CN, NO₂, C₁-C₆-Alkyloxy und CF₃ bestehenden Gruppe substituiert ist;
R₆ und R₇ unabhängig voneinander aus der aus H, Methyl, Ethyl, Propyl, Butyl, Isopropyl, Isobutyl, Cyclohexyl, Aryl, substituiertem Aryl, Heteroaryl und substituiertem Heteroaryl bestehenden Gruppe ausgewählt sind;
und deren pharmazeutisch unbedenkliche Salze;
wobei in den Verbindungen mit dem Begriff "substituiert" in dem Ausdruck "substituiertes Alkyl" das Vorhandensein eines oder mehrerer Substituenten ausgewählt aus der aus Halogen, CN, OH, NO₂, Amino, Aryl, Heterocyclyl, substituiertem Aryl, substituiertem Heterocyclyl, C₁-C₈-Alkyloxy, Aryloxy, substituiertem C₁-C₈-Alkyloxy, C₁-C₈-Alkylcarbonyl, C₁-C₈-Alkylcarboxyl, C₁-C₈-Alkylamino und Arylthio bestehenden Gruppe bezeichnet wird;
mit dem Begriff "substituiert" in dem Ausdruck "substituiertes Aryl" das Vorhandensein von 1 bis 4 Substituenten ausgewählt aus der aus Halogen, CN, OH, NO₂, Amino, C₁-C₈-Alkyl, Cycloalkyl, C₂-C₈-Alkenyl, C₂-C₈-Alkinyl, C₁-C₈-Alkoxy, Aryloxy, substituiertem C₁-C₈-Alkyloxy, C₁-C₈-Alkylcarbonyl, C₁-C₈-Alkylcarboxyl, C₁-C₈-Alkylamino und Arylthio bestehenden Gruppe bezeichnet wird;
mit dem Begriff "Heteroaryl" Aryl, das einen 4- bis 7gliedrigen monocyclischen heterocyclischen Ring oder einen multicyclischen Ring, der aus Kohlenstoffatomen und 1 bis 4 aus der aus N-, O-und S-Atomen bestehenden Gruppe ausgewählten Heteroatomen besteht, oder ein multicyclisches Ringsystem, in dem einer der oben erwähnten heterocyclischen Ringe mit einem Arylring kondensiert ist, umfaßt, bezeichnet wird;
mit dem Begriff "substituiert" in dem Ausdruck "substituiertes Heteroaryl" das Vorhandensein von 1 bis 4 Substituenten ausgewählt aus der aus Halogen, CN, OH, NO₂, Amino, C₁-C₈-Alkyl, substituiertem C₁-C₈-Alkyl, Cycloalkyl, C₂-C₈-Alkenyl, substituiertem C₂-C₈-Alkenyl, C₂-C₈-Alkinyl, substituiertem C₂-C₈-Alkinyl, C₁-C₈-Alkyloxy, Aryloxy, substituiertem C₁-C₈-Alkyloxy, C₁-C₈-Alkylcarbonyl, C₁-C₈-Alkylcarboxyl, C₁-C₈-Alkylamino und Arylthio bestehenden Gruppe bezeichnet wird;
der Begriff "substituiert" in den Ausdrücken "substituiertes Alkinyl" und "substituiertes Alkenyl" die gleichen Bedeutungen wie in dem Ausdruck "substituiertes Alkyl" hat;
mit dem Ausdruck "Heterocyclyl" ein 4- bis 7gliedriger monocyclischer heterocyclischer Ring oder ein multicyclischer Ring, der aus Kohlenstoffatomen und 1 bis 4 aus der aus N-, O-und S-Atomen bestehenden Gruppe ausgewählten Heteroatomen besteht oder ein multicyclisches Ringsystem, in dem einer der oben erwähnten heterocyclischen Ringe mit einem Arylring kondensiert ist, bezeichnet; und
der Begriff "substituiert" in dem Ausdruck "substituiertes Heterocyclyl" die gleichen Bedeutungen wie in dem Ausdruck "substituiertes Heteroaryl" hat.

2. Verbindungen der Formel: 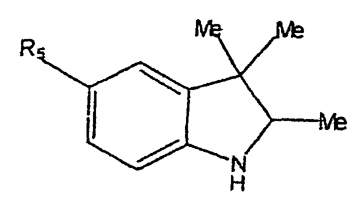 in welcher:
R₅ für (i) oder (ii) steht:
(i) ein substituierter Benzolring der Formel: 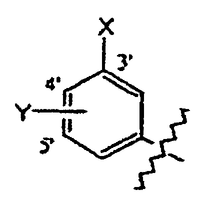
X ist aus der aus Halogen, CN, C₁-C₃-Alkyloxy, C₁-C₃-Alkyl, NO₂, C₁-C₃-Perfluoralkyl, einem 5gliedrigen heterocyclischen Ring, der 1 bis 3 Heteroatome in seinem Gerüst enthält, und Thio-C₁-C₃-alkoxy bestehenden Gruppe ausgewählt;
Y befindet sich in der 4'- oder 5'-Stellung und ist aus der aus H, Halogen, CN, NO₂, C₁-C₃-Alkyloxy, C₁-C₄-Alkyl und Thio-C₁-C₃-alkyl bestehenden Gruppe ausgewählt;
(ii)ein 5gliedriger heterocyclischer Ring der Formel: 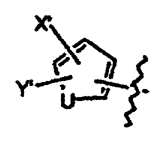 in welcher:
U für O, S oder NR₈ steht;
R₈ für H, C₁-C₃-Alkyl oder C₁-C₄-CO₂-Alkyl steht;
X' aus der aus Halogen, CN, NO₂, C₁-C₃-Alkyl und
C₁-C₃-Alkyloxy bestehenden Gruppe ausgewählt ist;
Y' aus der aus H, Halogen, CN, NO₂ und C₁-C₄-Alkyl bestehenden Gruppe ausgewählt ist;
wobei Halogen für F steht;
und deren pharmazeutisch unbedenkliche Salze.

3. Verbindungen, bei denen es sich um 4a-Methyl-6-(3-nitrophenyl)-2,3,4,4a,9,9a-hexahydro-1H-carbazol und deren pharmazeutisch unbedenkliche Salze handelt.

4. Verbindungen nach Anspruch 2, mit der Formel: 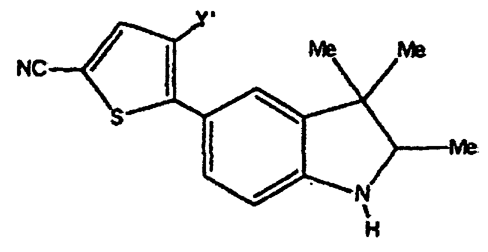 wobei Y' für H oder C₁-C₄-Alkyl steht.

5. Verbindungen nach Anspruch 1 oder 2, mit der Formel: 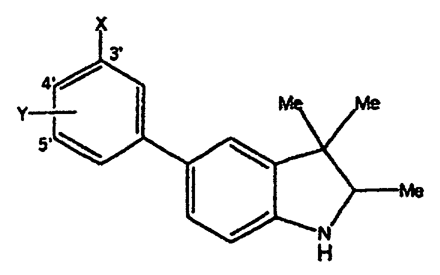 wobei:
X aus der aus Halogen, CN, C₁-C₃-Alkyloxy, C₁-C₃-Alkyl, NO₂, C₁-C₃-Perfluoralkyl, einem 5gliedrigen heterocyclischen Ring, der 1 bis 3 Heteroatome in seinem Gerüst enthält, und Thio-C₁-C₃-alkyloxy bestehenden Gruppe ausgewählt ist;
Y sich in der 4'- oder 5'-Stellung befindet und aus der aus H, Halogen, CN, NO₂, C₁-C₃-Alkyloxy, C₁-C₃-Alkyl und Thio-C₁-C₃-alkyl bestehenden Gruppe ausgewählt ist.

6. Verbindungen nach Anspruch 2, wobei X für CN oder NO₂ steht und Y für 5'-Fluor steht.

7. Verbindungen nach Anspruch 1 oder 2, mit der Formel: 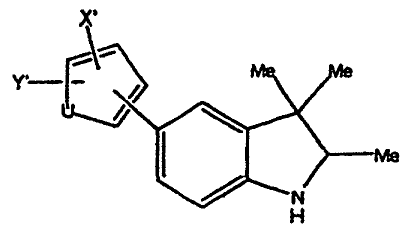 wobei:
U für O, S oder NR₈ steht;
R₈ für H oder C₁-C₃-Alkyl steht;
X' aus der aus Halogen, CN, NO₂, C₁-C₃-Alkyl und C₁-C₃-Alkyloxy bestehenden Gruppe ausgewählt ist;
Y' aus der aus H, Halogen, CN, NO₂ und C₁-C₃-Alkyl bestehenden Gruppe ausgewählt ist;
wobei Halogen für F steht.

8. Verbindungen nach einem der Ansprüche 1, 2, 4 oder 7, mit der Formel: 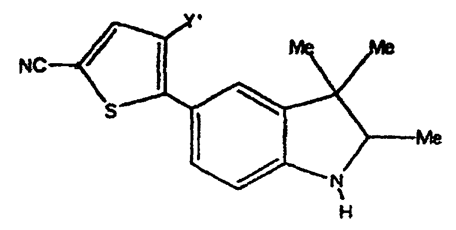 wobei Y' für H oder C₁-C₃-Alkyl steht.

9. Verbindung nach einem der Ansprüche 1, 2 oder 7, mit der Formel:

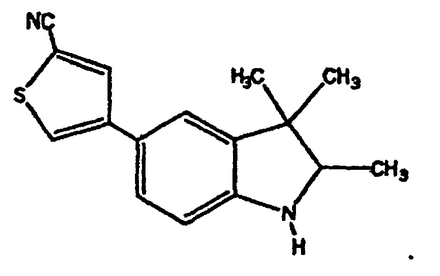

10. Verbindungen nach Anspruch 1, mit der Formel: 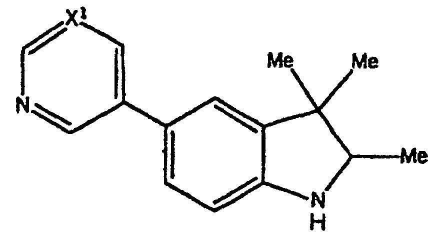 wobei:
X¹ für N oder CX² steht;
X² für Halogen, CN oder NO₂ steht.

11. Verbindungen nach Anspruch 1, bei denen es sich um 2,3,3-Trimethyl-5-(3-nitrophenyl)-2,3-dihydro-1H-indol, 2,3,3-Trimethyl-5-(3-nitrophenyl)-2,3-dihydro-1H-indol-hydrochlorid, (2-R)-2,3,3-Trimethyl-5-(3-nitrophenyl)-2,3-dihydro-1H-indol, (2-S)-2,3,3-Trimethyl-5-(3-nitrophenyl)-2,3-dihydro-1H-indol, 2,3,3-Diethyl-2-methyl-5-(3-nitrophenyl)-2,3-dihydro-1H-indol, 1,2-Dihydro-2-methyl-5-(3-nitrophenyl)spiro[cyclohexan-1,3-[3H]indol], 3,3-Dimethyl-5-(3-nitrophenyl)-2,3-dihydro-1H-indol, 5'-(3-Chlorphenyl)-1',2'-dihydrospiro[cyclohexan-1,3'-[3H]indol] und 3-(2',3',3'-Trimethyl-2',3-dihydro-1H-indol-5'-yl)benzonitril und deren pharmazeutisch unbedenkliche Salze handelt.

12. Pharmazeutische Zusammensetzung, enthaltend eine pharmazeutisch wirksame Menge einer Verbindung nach einem der Ansprüche 1 bis 11 und einen pharmazeutisch unbedenklichen Träger oder Hilfsstoff.

13. Verwendung einer Verbindung nach einem der Ansprüche 1 bis 11 oder eines unbedenklichen Salzes davon zur Herstellung eines Medikaments.

14. Verwendung einer Verbindung nach einem der Ansprüche 1 bis 11 oder eines unbedenklichen Salzes davon zur Herstellung eines Medikaments, das einem Säugetier als Empfängnisverhütungsmittel verabreicht werden kann.

15. Verwendung einer Verbindung nach einem der Ansprüche 1 bis 11 oder eines pharmazeutisch unbedenklichen Salzes davon zur Herstellung eines Medikaments, das einem Säugetier zur Behandlung oder Prävention einer gutartigen oder bösartigen neoplastischen Erkrankung verabreicht werden kann.

16. Verwendung nach Anspruch 15, wobei die gutartige oder bösartige neoplastische Erkrankung aus der aus Fibromen des Uterusmyometriums, Endometriose, gutartiger Prostatavergrößerung, Karzinomen und Adenokarzinomen des Endometriums, der Eierstöcke, der Brust, des Kolons, der Prostata und der Hypophyse, Meningioma und anderen hormonabhängigen Tumoren bestehenden Gruppe ausgewählt ist.

17. Verwendung einer Verbindung nach einem der Ansprüche 1 bis 11 oder eines unbedenklichen Salzes davon zur Herstellung eines Medikaments, das man einem Säugetier zur Hormonersatztherapie verabreichen kann.

## Revendications

1. Composé de formule : 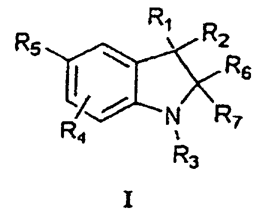 dans laquelle :
R₁ et R₂ sont choisis indépendamment dans le groupe constitué par les radicaux H, alkyle en C₁ à C₈, alkyle en C₁ à C₈ substitué, OH, O(alkyle en C₁ à C₈), O(alkyle substitué en C₁ à C₈), O(acétyle), aryle, aryle substitué, hétéroaryle, hétéroaryle substitué, alkyl(en C₁ à C₈)aryle, alkyl(en C₁ à C₈)hétéroaryle, 1-propynyle et 3-propynyle ;
ou bien R₁ et R₂ sont reliés pour former un noyau comprenant -CH₂(CH₂)ₙCH₂-, -CH₂CH₂C(CH₃)₂CH₂CH₂-, -O(CH₂)ₘCH₂-, -O(CH₂)ₚO-, -CH₂CH₂OCH₂CH₂-, -CH₂CH₂N(alkyle en C₁ à C₈)CH₂CH₂- ou -CH₂CH₂NHCH₂CH₂- ;
n est un nombre entier de 0 à 5 ;
m est un nombre entier de 1 à 4 ;
p est un nombre entier de 1 à 4 ;
ou bien R₁ et R₂ constituent ensemble une double liaison à C(CH₃)₂, C(cycloalkyle en C₃ à C₆), O ou C(cycloéther) ;
où ledit cycloéther est choisi dans le groupe constitué par les radicaux tétrahydrofuranyle et hexahydropyranyle ;
R₃ représente un radical H, OH, NH₂, alkyle en C₁ à C₆, alkyle en C₁ à C₆ substitué, alcényle en C₃ à C₆, alcynyle en C₂ à C₈, alcynyle en C₂ à C₈ substitué ou COR^{A} ;
R^{A} représente un radical H, alkyle en C₁ à C₃, alkyle en C₁ à C₃ substitué, alkyloxy en C₁ à C₃, alkyloxy en C₁ à C₃ substitué, aminoalkyle en C₁ à C₃, ou aminoalkyle en C₁ à C₃ substitué;
R₄ représente un radical H, halogéno, CN, NH₂, alkyle en C₁ à C₆, alkyle en C₁ à C₆ substitué, alkyloxy en C₁ à C₆, alkyloxy en C₁ à C₆ substitué, aminoalkyle en C₁ à C₆ ou aminoalkyle en C₁ à C₆ substitué ;
R₅ est choisi dans le groupe constitué par a), b) et c) :
a) un noyau benzénique substitué de formule: 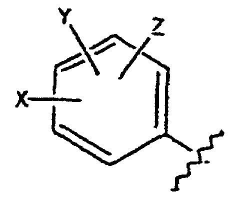
X est choisi dans le groupe constitué par les radicaux halogéno, OH, CN, alkyle en C₁ à C₃, alkyle en C₁ à C₃ substitué, alkyloxy en C₁ à C₃, alkyloxy en C₁ à C₃ substitué, thioalkyle en C₁ à C₃, thioalkyle en C₁ à C₃ substitué, S(O)alkyle en C₁ à C₈, S(O)₂alkyle en C₁ à C₈, aminoalkyle en C₁ à C₃, aminoalkyle en C₁ à C₃ substitué, NO₂, perfluoroalkyle en C₁ à C₃, un noyau hétérocyclique à 5 ou 6 chaînons contenant, dans sa chaîne principale, 1 à 3 hétéroatomes, COR^{B}, OCOR^{B} et NR^{C}COR^{B} ;
R^{B} représente un radical H, alkyle en C₁ à C₃, alkyle en C₁ à C₃ substitué, aryle, aryle substitué, alkyloxy en C₁ à C₃, alkyloxy en C₁ à C₃ substitué, aminoalkyle en C₁ à C₃ ou aminoalkyle en C₁ à C₃ substitué ;
R^{c} représente un radical H, alkyle en C₁ à C₃ ou alkyle en C₁ à C₃ substitué ;
Y et Z sont choisis indépendamment dans le groupe constitué par les radicaux H, halogéno, CN, NO₂, alkyloxy en C₁ à C₃, alkyle en C₁ à C₃ et thioalkyle en C₁ à C₃ ;
b) un noyau à cinq ou six chaînons comportant, dans sa chaîne principale, 1, 2 ou 3 hétéroatomes choisis dans le groupe constitué par O, S, SO, SO₂ et NR₈ et contenant un ou deux substituants indépendants choisis dans le groupe constitué par les radicaux H, halogéno, CN, NO₂, alkyle en C₁ à C₃, alkyloxy en C₁ à C₃, aminoalkyle en C₁ à C₃, COR^{D} et NR^{E}COR^{D} ;
R^{D} représente un radical H, alkyle en C₁ à C₃, alkyle C₁ à C₃ substitué, aryle, aryle substitué, alkyloxy en C₁ à C₃, alkyloxy en C₁ à C₃ substitué, aminoalkyle en C₁ à C₃ ou aminoalkyle en C₁ à C₃ substitué ;
R^{E} représente un radical H, alkyle en C₁ à C₃ ou alkyle en C₁ à C₃ substitué ;
R₈ représente un radical H ou alkyle en C₁ à C₃ ; et
c) un groupement indol-4-yle, indol-7-yle ; ou benzo-2-thiophène, le groupement étant éventuellement substitué par 1 à 3 substituants choisis dans le groupe constitué par les radicaux halogéno, alkyle en C₁ à C₆, CN, NO₂, alkyloxy en C₁ à C₆ et CF₃ ;
R₆ et R₇ sont choisis indépendamment dans le groupe constitué par les radicaux H, méthyle, éthyle, propyle, butyle, iso-propyle, iso-butyle, cyclohexyle, aryle, aryle substitué, hétéroaryle et hétéroaryle substitué ;
ou sel pharmaceutiquement acceptable de ce composé ;
dans ces composés, le terme "substitué", dans l'expression "alkyle substitué", désigne la présence d'un ou plusieurs substituants choisis dans le groupe constitué par les radicaux halogéno, CN, OH, NO₂, amino, aryle, hétérocyclique, aryle substitué, hétérocyclique substitué, alkyloxy en C₁ à C₈, aryloxy, alkyloxy en C₁ à C₈ substitué, alkyl(en C₁ à C₈)carbonyle, alkyl(en C₁ à C₈)carboxy, alkyl(en C₁ à C₈)amino et arylthio ;
le terme "substitué", dans l'expression "aryle substitué", désigne la présence de 1 à 4 substituants choisis dans le groupe constitué par les radicaux halogéno, CN, OH, NO₂, amino, alkyle en C₁ à C₈, cycloalkyle, alcényle en C₂ à C₈, alcynyle en C₂ à C₈, alcoxy en C₁ à C₈, aryloxy, alkyloxy en C₁ à C₈ substitué, alkyl(en C₁ à C₈)carbonyle, alkyl(en C₁ à C₈)carboxy, alkyl(en C₁ à C₈)amino et arylthio ;
le terme "hétéroaryle" désigne un radical aryle qui comprend un noyau hétérocyclique monocyclique à 4 - 7 chaînons ou un noyau multicyclique constitué d'atomes de carbone et de 1 à 4 hétéroatomes choisis dans le groupe constitué par les atomes N, O et S ou un système à noyau multicyclique dans lequel n'importe lequel des noyaux hétérocycliques indiqués ci-dessus est condensé à un noyau aryle ;
le terme "substitué", dans l'expression "hétéroaryle substitué", désigne la présence de 1 à 4 substituants choisis dans le groupe constitué par les radicaux halogéno, CN, OH, NO₂, amino, alkyle en C₁ à C₈, alkyle en C₁ à C₈ substitué, cycloalkyle, alcényle en C₂ à C₈, alcényle en C₂ à C₈ substitué, alcynyle en C₂ à C₈, alcynyle en C₂ à C₈ substitué, alkyloxy en C₁ à C₈, aryloxy, alkyloxy en C₁ à C₈ substitué, alkyl(en C₁ à C₈)carbonyle, alkyl(en C₁ à C₈)carboxy, alkyl(en C₁ à C₈)amino et arylthio ;
le terme "substitué", dans les expressions "alcynyle substitué" et "alcényle substitué", a les mêmes significations que dans l'expression "alkyle substitué" ;
le terme "hétérocyclique" désigne un noyau hétérocyclique monocyclique à 4 - 7 chaînons ou un noyau multicyclique constitué d'atomes de carbone et de 1 à 4 hétéroatomes choisis dans le groupe constitué par les atomes N, O et S, ou un système à noyau multicyclique dans lequel n'importe lequel des noyaux hétérocycliques ci-dessus est condensé à un noyau aryle ; et
le terme "substitué", dans l'expression "hétérocyclique substitué", a les mêmes significations que dans l'expression "hétéroaryle substitué".

2. Composé de formule: 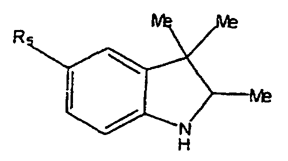 dans laquelle :
R₅ est (i) ou (ii):
(i)un noyau benzénique substitué de formule: 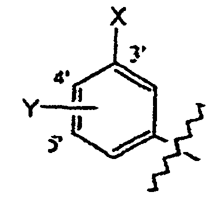
X est choisi dans le groupe constitué par les radicaux halogéno, CN, alkyloxy en C₁ à C₃, alkyle en C₁ à C₃, NO₂, perfluoroalkyle en C₁ à C₃, un noyau hétérocyclique à 5 chaînons contenant, dans sa chaîne principale, 1 à 3 hétéroatomes, et thioalcoxy en C₁ à C₃ ;
Y est en position 4' ou 5' et est choisi dans le groupe constitué par les radicaux H, halogéno, CN, NO₂, alkyloxy en C₁ à C₃, alkyle en C₁ à C₄ et thioalkyle en C₁ à C₃ ;
(ii) un noyau hétérocyclique à 5 chaînons de formule: 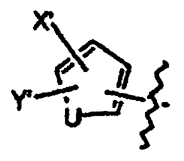 dans laquelle :
U représente O, S ou NR₈ ;
R₈ représente un radical H, alkyle en C₁ à C₃ ou CO₂-alkyle en C₁ à C₄ ;
X' est choisi dans le groupe constitué par les radicaux halogéno, CN, NO₂, alkyle en C₁ à C₃ et alkyloxy en C₁ à C₃ ;
Y' est choisi dans le groupe constitué par les radicaux H, halogéno, CN, NO₂ et alkyle en C₁ à C₄ ;
où ledit radical halogéno est F ;
ou sel pharmaceutiquement acceptable de ce composé.

3. Composé qui est le 4a-méthyl-6-(3-nitrophényl)-2,3,4,4a,9,9a-hexahydro-1H-carbazole ou un sel pharmaceutiquement acceptable de ce composé.

4. Composé selon la revendication 2 de formule : 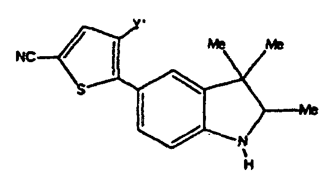 dans laquelle Y' représente un radical H ou alkyle en C₁ à C₄.

5. Composé selon la revendication 1 ou 2 de formule: 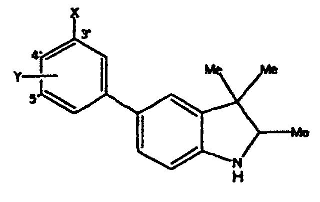 dans laquelle :
X est choisi dans le groupe constitué par les radicaux halogéno, CN, alkyloxy en C₁ à C₃, alkyle en C₁ à C₃, NO₂, perfluoroalkyle en C₁ à C₃, un noyau hétérocyclique à 5 chaînons contenant, dans sa chaîne principale, 1 à 3 hétéroatomes, et thioalkyloxy en C₁ à C₃ ;
Y est en position 4' ou 5' et est choisi dans le groupe constitué par les radicaux H, halogéno, CN, NO₂, alkyloxy en C₁ à C₃, alkyle en C₁ à C₃ et thioalkyle en C₁ à C₃.

6. Composé selon la revendication 2, dans lequel X est un radical CN ou NO₂ et Y est un radical 5'-fluoro.

7. Composé selon la revendication 1 ou 2 de formule : 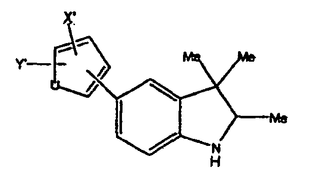 dans laquelle :
U représente O, S ou NR₈ ;
R₈ représente H ou alkyle en C₁ à C₃ ;
X' est choisi dans le groupe constitué par les radicaux halogéno, CN, NO₂, alkyle en C₁ à C₃ et alkyloxy en C₁ à C₃ ;
Y' est choisi dans le groupe constitué par les radicaux H, halogéno, CN, NO₂ et alkyle en C₁ à C₃ ;
où ledit radical halogéno est F.

8. Composé selon l'une quelconque des revendications 1, 2, 4 ou 7 de formule: 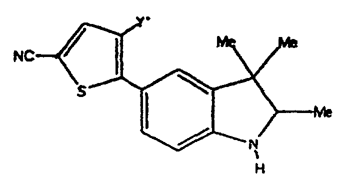 dans laquelle Y' représente un radical H ou alkyle en C₁ à C₃.

9. Composé selon l'une quelconque des revendications 1, 2 ou 7, de formule:

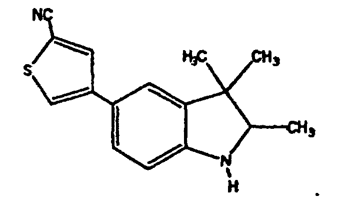

10. Composé selon la revendication 1, de formule 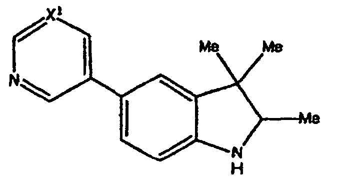 dans laquelle :
X¹ représente N ou CX² ;
X² représente un radical halogéno, CN ou NO₂.

11. Composé selon la revendication 1, qui est le 2,3,3-triméthyl-5-(3-nitrophényl)-2,3-dihydro-1H-indole, le chlorhydrate de 2,3,3-triméthyl-5-(3-nitrophényl)-2,3-dihydro-lH-indole, le (2-R)-2,3,3-triméthyl-5-(3-nitrophényl)-2,3-dihydro-1H-indole, le (2-S)-2,3,3-triméthyl-5-(3-nitrophényl)-2,3-dihydro-1H-indole, le 2,3,3-diéthyl-2-méthyl-5-(3-nitrophényl)-2,3-dihydro-1H-indole, le 1,2-dihydro-2-méthyl-5-(3-nitrophényl)spiro[cyclohexane-1,3-[3H]indole], le 3,3-diméthyl-5-(3-nitrophényl)-2,3-dihydro-1H-indole, le 5'-(3-chlorophényl)-1',2'-dihydrospiro[cyclohexane-1,3'-[3H]indole] et le 3-(2',3',3'-triméthyl-2',3-dihydro-1H-indol-5'-yl)benzonitrile, ou un sel pharmaceutiquement acceptable de ces composés.

12. Composition pharmaceutique comprenant une quantité efficace sur le plan pharmaceutique d'un composé selon l'une quelconque des revendications 1 à 11 et un véhicule ou excipient pharmaceutiquement acceptable.

13. Utilisation d'un composé selon les revendications 1 à 11, ou dudit sel acceptable de ce composé, pour préparer un médicament.

14. Utilisation du composé selon l'une quelconque des revendications 1 à 11, or dudit sel acceptable, pour préparer un médicament utile à administrer à un sujet mammalien comme contraceptif.

15. Utilisation du composé selon l'une quelconque des revendications 1 à 11, ou dudit sel acceptable, pour préparer un médicament utile à administrer à un sujet mammalien dans le but de traiter ou de prévenir une maladie néoplasique bénigne ou maligne.

16. Utilisation selon la revendication 15, dans laquelle la maladie néoplasique bénigne ou maligne est choisie dans le groupe constitué par les fibroïdes du myomètre utérin, l'endométriose, l'hypertrophie bénigne de la prostate, les carcinomes et adénocarcinomes de l'endomètre, l'ovaire, du sein, du côlon, de la prostate, de l'hypophyse, le méningiome et d'autres tumeurs hormono-dépendantes.

17. Utilisation du composé selon l'une quelconque des revendications 1 à 11, ou dudit sel acceptable, pour préparer un médicament à administrer à un sujet mammalien destiné à une hormonothérapie substitutive.
